# EUROPEAN PATENT APPLICATION

(11) **EP 4 190 301 A1**
(43) Date of publication of application: **07.06.2023**
(21) Application number: 21849270.0
(22) Date of filing: 28.07.2021
(51) Int. Cl.: A61H 15/00

(54) **MASSAGE APPARATUS FOR MEASURING BIO-SIGNALS**

(30) Priority: 30.07.2020 KR 20200095223; 30.07.2020 KR 20210008592; 23.10.2020 KR 20200138059; 25.01.2021 KR 20210010176
(71) Applicant: Bodyfriend Co., Ltd., Seoul 06302 (KR)
(72) Inventor: KIM, Do Hyun, Seoul 06302 (KR); KIM, Jin Hwan, Seoul 06302 (KR); LIM, Sung Gu, Seoul 06302 (KR); JEON, Chul Jin, Seoul 06302 (KR); CHO, Soo Hyun, Seoul 06302 (KR)
(74) Representative: Viering, Jentschura & Partner mbB Patent- und Rechtsanwälte
(86) International application number: PCT/KR2021/009790
(87) International publication number: WO 2022/025622

(57) **Abstract**

The present invention relates to a massage apparatus comprising: a first bio-signal measurement unit which is formed in an arm massage unit and includes at least one electrode positioned at a portion where the palm of a user is placed, and which is movable back and forth on the basis of the position of the hand of the user; a second bio-signal measurement unit which is formed in a foot massage unit and includes at least one electrode positioned at the rear portion of the ankle of the user, and is movable back and forth; and a control unit for acquiring information on the physical condition of the user on the basis of bio-signals obtained from the first bio-signal measurement unit and the second bio-signal measurement unit.

## Description

### Technical Field

The present invention relates to a massage apparatus, and more specifically, to a massage apparatus for measuring bio-signals.

### Background Art

Massage is a medical adjuvant therapy for controlling the modulation of the body of a user helping blood circulation, and relieving fatigue of the user by applying various types of mechanical stimulation to a portion of the body, such as massaging, pressing, pulling, tapping, or moving a portion of the body of the user, helping blood circulation.

The growth in a massage demand caused an increase in demand for massage apparatuses or massage devices, which provide an artificial massage function, because of economic feasibility and temporal reasons. That is, as the demand for relieving fatigue or stress is increased while loosening the agglomerated muscles through massage, various mechanical massage apparatuses for time and cost efficiencies are being released. Any type of instruments, devices, or apparatuses for performing massage without a massagist is referred to as a massage apparatus.

Furthermore, the demands of users for a massage apparatus capable of relieving fatigue or stress and promoting health through massage are increasing.

Therefore, studies for providing a massage apparatus capable of promoting health of users have continued.

Korean Patent No. 10-0941919 discloses a massage apparatus for providing massage capable of promoting a user's health.

### Disclosure

### Technical Problem

Accordingly, the present invention has been made in view of the above-mentioned problems occurring in the related art, and it is an object of the present invention to provide a massage apparatus for measuring bio-signals.

Technical objects to be achieved by the present invention are not limited to the above-described objects and other technical objects that have not been described will be evidently understood by those skilled in the art from the following description.

### Technical Solution

To accomplish the above-mentioned objects, according to the present invention, there is provided a massage apparatus for measuring bio-signals including: a first bio-signal measurement unit which is formed in an arm massage unit, is located at a portion on which a user's palm is put, is movable forward and backward on the basis of the position of the hand of the user, and includes at least one electrode; a second bio-signal measurement unit which is formed in a foot massage unit, is located at a portion corresponding to the back of the ankle of the user, is movable forward and backward and includes at least one electrode; and a control unit for acquiring information on the user's physical condition on the basis of bio-signals obtained from the first bio-signal measurement unit and the second bio-signal measurement unit.

The first bio-signal measurement unit of the massage apparatus according to the present invention includes: a lower cover put on a housing of the arm massage unit; at least two magnets fixed on the lower cover and facing a plate; an upper cover covering the lower cover to protect various components disposed on the lower cover, coupled to the lower cover, and including a palm support having an upward convex shape so that the user can comfortably put the hand thereon; a first bio-signal measurement electrode inserted into a hole formed in the upper cover; and the plate made of a metal material, fixed on a cover sheet of the arm massage unit, and located on the upper cover.

The first bio-signal measurement unit of the massage apparatus according to the present invention further includes: a plurality of LEDs for treatment which are fixed on the lower cover to emit light based on a control signal of the control unit, and wherein a portion of the upper cover is transparent to transmit light of the plurality of LEDs to the user's hand.

The first bio-signal measurement unit of the massage apparatus according to the present invention further includes: a heating unit support fixed on the lower cover, located below a heating unit to maintain the shape of the heating unit, and having an upwardly convex shape; and the heating unit fixed on the heating unit support, formed along the convex surface of the heating unit support, and emitting heat on the basis of the control signal of the control unit.

The heating unit of the massage apparatus according to the present invention is located on the plurality of LEDs for treatment, and includes at least one slit not to hide light emitted from the plurality of LEDs for treatment.

Guide rails are formed on the plate in the back-and-forth direction, and the lower cover moves back and forth along the guide rails.

The upper cover of the massage apparatus according to the present invention includes an upper cover fixing portion which is formed along the circumference of the palm support of the upper cover and is flat, and at least a portion of the upper cover fixing portion is positioned under a cover sheet of the arm massage unit to prevent the upper cover from being separated from the cover sheet of the arm massage unit.

The second bio-signal measurement unit of the massage apparatus according to the present invention includes: a leg support formed on the housing of the foot massage unit to be located at the back of the leg of the user; a foot holder coupled to the front of the leg support to support the user's leg, and having a concave shape; an elastic layer attached to the front of the foot holder; and a second bio-signal measurement electrode inserted into the hole formed in the cover layer.

The leg support of the massage apparatus according to the present invention has a coupling groove into which a coupling protrusion, a spring, and a guide pin included in the foot holder are inserted. The coupling protrusion of the foot holder has a groove so that a guide ring can be inserted into the groove. The leg support and the foot holder are coupled to each other such that the guide pin is inserted into the spring, the guide pin is inserted into the guide ring, the coupling protrusion is inserted into the coupling groove, and the spring is compressed by the guide ring to push the guide ring forward.

The massage apparatus according to the present invention includes two first bio-signal measurement units wherein each of the two first bio-signal measurement units includes two first bio-signal measurement electrodes, the massage apparatus comprises two second bio-signal measurement units wherein each of the two second bio-signal measurement units includes two second bio-signal measurement electrodes, and the control unit measures bio-signals of the user by using at least two out of a total of four first bio-signal measurement electrodes and at least two out of a total of four second bio-signal measurement electrodes.

The control unit measures the user's bio-signals at a predetermined cycle by using the first bio-signal measurement unit and the second bio-signal measurement unit while the massage apparatus provides a massage to the user according to a massage mode.

The control unit of the massage apparatus according to the present invention simultaneously measures body composition information and electrocardiogram information of the user by using the first bio-signal measurement unit and the second bio-signal measurement unit.

Furthermore, a program for realizing an operating method of the massage apparatus described above may be recorded in a computer-readable recording medium.

### Advantageous Effects

The massage apparatus according to an embodiment of the present invention can measure a user's bio-signals through the bio-signal measurement module. In addition, the massage apparatus according to an embodiment of the present invention can provide information on the user's body and provide a massage customized to a user.

The effects obtainable by the container collecting apparatus according to the present invention are not limited to the above-mentioned effects and further effects not described above will be clearly understood by those skilled in the art.

### Description of Drawings

FIG. 1 is a view for depicting a massage apparatus according to an embodiment of the present invention.
FIG. 2 is a view for depicting a main frame according to an embodiment of the present invention.
FIG. 3 is a view illustrating a massage apparatus according to an embodiment of the present invention.
FIG. 4 is a view illustrating an external device capable of communicating with a massage apparatus according to an embodiment of the present invention.
FIG. 5 is a view for depicting a first bio-signal measurement unit according to an embodiment of the present invention.
FIG. 6 is a view for depicting a second bio-signal measurement unit according to an embodiment of the present invention.
FIG. 7 is a view for depicting a method of measuring body composition by a user through the second bio-signal measurement unit according to an embodiment of the present invention.
FIG. 8 is a perspective view of a first bio-signal measurement unit according to an embodiment of the present invention.
FIG. 9 is a view illustrating components included in the first bio-signal measurement unit according to an embodiment of the present invention.
FIG. 10 is a view illustrating a first bio-signal measurement unit according to an embodiment of the present invention.
FIG. 11 is a view of a second bio-signal measurement unit according to an embodiment of the present invention.
FIG. 12 is a view illustrating a second bio-signal measurement unit according to an embodiment of the present invention.
FIG. 13 is a view illustrating a second bio-signal measurement unit according to an embodiment of the present invention.
FIG. 14 is a view illustrating a second bio-signal measurement unit according to an embodiment of the present invention.
FIG. 15 is a view for depicting an example that a posture of the massage apparatus is adjusted to measure a bio-signal according to an embodiment of the present invention.
FIG. 16 is a view for depicting a first bio-signal measurement unit according to an embodiment of the present invention.
FIG. 17 is a view illustrating a magnet according to an embodiment of the present invention.
FIG. 18 is a perspective view of a first bio-signal measurement unit according to an embodiment of the present invention.
FIG. 19 is a view illustrating components included in the first bio-signal measurement unit according to an embodiment of the present invention.
FIG. 20 is a view illustrating a first bio-signal measurement unit according to an embodiment of the present invention.
FIG. 21 is a view illustrating a first bio-signal measurement unit according to an embodiment of the present invention.
FIG. 22 is a view illustrating a third bio-signal measurement unit according to an embodiment of the present invention.
FIG. 23 is a view illustrating a second bio-signal measurement unit and a fourth bio-signal measurement unit according to an embodiment of the present invention.
FIG. 24 is a view illustrating a fourth bio-signal measurement unit according to an embodiment of the present invention.
FIG. 25 is a view illustrating a second bio-signal measurement unit according to an embodiment of the present invention.
FIG. 26 is a view for depicting functions of a functional button according to an embodiment of the present invention.

### Best Mode

Provided is a massage apparatus including: a first bio-signal measurement unit which is formed in an arm massage unit, is located at a portion on which a user's palm is put, is movable forward and backward on the basis of the position of the hand of the user, and includes at least one electrode; a second bio-signal measurement unit which is formed in a foot massage unit, is located at a portion corresponding to the back of the ankle of the user, is movable forward and backward and includes at least one electrode; and a control unit for acquiring information on the user's physical condition on the basis of bio-signals obtained from the first bio-signal measurement unit and the second bio-signal measurement unit.

### Mode for Invention

Advantages and features of the present invention and methods accomplishing the advantages and features will become apparent from the following detailed description of exemplary embodiments with reference to the accompanying drawings. However, the present invention is not limited to exemplary embodiment disclosed herein but will be implemented in various forms. The exemplary embodiments are provided so that the present invention is completely disclosed, and a person of ordinary skilled in the art can fully understand the scope of the present invention.

Terms used herein will be briefly described, and the disclosed embodiments will be described in detail.

The terms including descriptive or technical terms which are used herein should be construed as having meanings that are obvious to one of ordinary skill in the art. However, the terms may have different meanings according to an intention of one of ordinary skill in the art, precedent cases, or the appearance of new technologies. Also, some terms may be arbitrarily selected by the applicant, and in this case, the meaning of the selected terms will be described in detail in the detailed description of the invention. Thus, the terms used herein have to be defined based on the meaning of the terms together with the description throughout the specification.

The singular forms of the components may be understood into the plural forms unless otherwise specifically stated in the context. Furthermore, the plural forms may include singular forms unless otherwise specifically stated in the context.

In the entire specification of the present invention, when a certain part "includes" a certain component, other components are not excluded unless explicitly described otherwise, and other components may in fact be included.

The term, "unit", used in the present invention means a software element or a hardware element, and the "unit" performs some roles. However, the term, "unit", is not limited to software or hardware. The "unit" may be configured in an addressable storage medium or may be configured to play one or more processors. Therefore, as an example, a "unit" includes elements, such as software elements, object-oriented software elements, class elements, and task elements, processes, functions, attributes, procedures, subroutines, segments of program codes, drivers, firmware, microcode, circuits, data, databases, data structures, tables, arrays, and variables. Functions provided within the elements and "units" may be combined with a smaller number of elements and "units" or may be further divided into additional elements and "units".

According to an embodiment of the present invention, the "unit" may be implemented as a processor and a memory. The term "processor" should be interpreted broadly to encompass a general-purpose processor, a central processing unit (CPU), a microprocessor, a digital signal processor (DSP), a controller, a microcontroller, a state machine, and the like. In some environments, a "processor" may refer to an application specific integrated circuit (ASIC), a programmable logic device (PLD), a field programmable gate array (FPGA), or the like. The term "processor" may refer to a combination of processing devices, such as a combination of a DSP genome and a microprocessor, a combination of a plurality of microprocessors, a combination of one or more microprocessors in combination with a DSP couple core, or a combination of any other such configurations.

The term "memory" should be interpreted broadly to include any electronic component capable of storing electronic information. The term memory may refer to various types of processor-readable media such as random access memory (RAM), read-only memory (ROM), non-volatile random access memory (NVRAM), programmable read-only memory (PROM), erase-programmable read-only memory (EPROM ACL), electrically erasable PROM (EEPROM), flash memory, magnetic or optical data storage, registers, and the like. Memory is referred to as being in electronic communication with the processor if the processor can read information from, and/or write information to, the memory. The memory integrated in the processor is in electronic communication with the processor.

In the present specification, an actuator refers to a configuration capable of providing a driving force. For example, the actuator may include a motor, a linear motor, an electronic motor, a DC motor, an AC motor, a linear actuator, an electric actuator, and the like, but is not limited thereto.

In the present specification, according to an embodiment, a massage apparatus may refer to a massage apparatus including a body massage unit and a leg massage unit. In addition, according to another embodiment, a body massage unit 2100 and a leg massage unit 2300 may be provided as separate devices (for example, a body massage apparatus and a leg massage apparatus), and the massage apparatus may refer to a body massage device or a leg massage device.

Hereinafter, embodiments of the present invention will be described in detail with reference to the accompanying drawings so as to be easily carried out by a person skilled in the art to which the present invention pertains. In addition, in order to clearly describe the present invention, portions irrelevant to the description will be omitted.

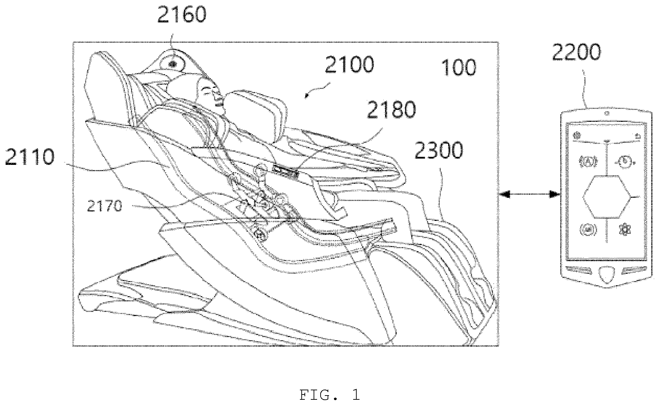

FIG. 1 is a view for depicting a massage apparatus according to an embodiment of the present invention.

The massage apparatus 100 according to an embodiment of the present invention may have an area for accommodating at least a portion of the body of a user, and include a body massage unit 2100 for massaging the body of the user, and a leg massage unit 2300 for massaging the user's legs.

The body massage unit 2100 may provide massage to at least a portion of the body of the user. The body massage unit 2100 may include a massage module 2170 for providing a massage function to at least a portion of the body of the user, an audio output module 2160 for providing an audio output of an arbitrary form to the user, a main frame 2110 constituting a framework of the body massage unit 2100, and a user input unit 2180 for receiving any form of input from the user.

The components included in the body massage unit 2100 described above are merely exemplary embodiments, and the body massage unit 2100 may include various configurations besides the above described configuration.

In addition, the shape and structure of the massage apparatus 100 illustrated in FIG. 1 are merely exemplary, and various types of massage apparatuses 100 may also be included within the scope of the present invention unless departing from the scope of the present invention defined by the claims of the present invention.

The body massage unit 2100 may form a space for accommodating the user. The body massage unit 2100 may have a space corresponding to the shape of the body of the user. For example, as illustrated in FIG. 1, the body massage unit 2100 may be implemented as a seating type capable of accommodating the whole body or a portion of the body of the user.

A portion of the body massage unit 2100, which gets in contact with the ground, may include any material or any member (for example, an anti-slip pad or the like) for increasing a frictional force, and may include a wheel for enhancing mobility of the massage apparatus 100.

At least a portion of the body massage unit 2100 may slide. For example, in a case in which the body massage unit 2100 starts massage, at least a portion of the body massage unit 2100 may slide forward. In addition, the body massage unit 2100 may be tilted backward. As a result, the body massage unit 2100 may provide massage while being tilted backward.

According to an embodiment of the present invention, the massage apparatus 100 may include at least one air cell (not illustrated). The air cell may be located at a shoulder portion, a pelvis portion, arm massage units, a leg massage unit 2300, and the like, but is not limited thereto, and may be disposed in various portions of the massage apparatus 100.

The massage apparatus 100 may include an air supply unit, and the air supply unit may inflate the air cell by supplying air to the air cell. The air supply unit may be located inside the body massage unit 2100 and may be located in the leg massage unit 2300. Additionally, the air supply unit may be located outside the massage apparatus 100.

The leg massage unit 2300 may provide a leg massage to the user. For example, the leg massage unit 2300 may include a calf massage unit for massaging the calves of the user and/or a foot massage unit for massaging the user's feet.

The leg massage unit 2300 may adjust the length according to the body characteristics of the user. For example, in a case in which a user who is tall uses the massage apparatus 100, it is necessary to lengthen the leg massage unit 2300 since the user has long calves. Moreover, in a case in which a user who is short uses the massage apparatus 100, it is necessary to shorten the leg massage unit 2300 since the user has short calves.

The massage module 2170 may be provided inside the body massage unit 2100 to provide a mechanical stimulation to the user accommodated in the body massage unit 2100. As illustrated in FIG. 1, the massage module 2170 may move along a main frame 2110 provided inside the body massage unit 2100.

For example, a rack gear may be provided in the main frame 2110 of the body massage unit 2100. The massage module 2170 may provide dynamic stimulation to various parts of the body of the user while moving along the rack gear. The massage module 2170 may include a ball massage unit or a roller massage unit, but is not limited thereto.

The main frame 2110 constitutes a frame of an internal configuration of the body massage unit 2100, and may be formed of a metal material, a plastic material, or the like. For example, the main frame 2110 may be made of iron, an alloy, steel, or the like, but is not limited thereto, and may be made with various rigid materials.

According to an embodiment of the present invention, the massage apparatus 100 may include an audio output module 2160. The audio output module 2160 may be provided at various positions. For example, the audio output module 2160 may include a plurality of output units, such as an upper audio output unit disposed on an upper end of a seat portion getting in contact with the user, a front audio output unit attached to a front end of the left and right arm massage units of the seat portion, and/or a rear audio output units attached to rear ends of the arm massage units, but is not limited thereto. In this case, the audio output module 2160 may provide a stereoscopic sound, such as a 5.1 channel, but is not limited thereto.

According to an embodiment of the disclosure, the user may control the massage apparatus 100 by using a massage apparatus control device 2200. The massage apparatus control device 2200 may be connected to the massage apparatus 100 through wired communication and/or wireless communication.

The massage apparatus control device 2200 may include a remote controller, a cellular phone, a personal digital assistant (PDA), and the like, but is not limited thereto, and may include various electronic devices connectable to the massage apparatus 100 through wired or wireless communication.

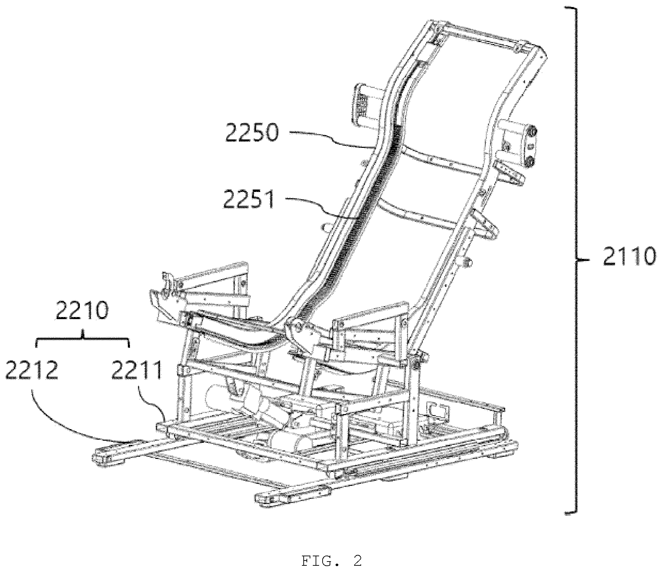

FIG. 2 is a view for depicting a main frame according to an embodiment of the present invention.

According to an embodiment of the present invention, the main frame 2110 may include an upper frame 2250 on which the massage module 2170 is disposed, and a base frame 2210 supporting the upper frame 2250.

At least a portion of the upper frame 2250 may be provided with a rack gear 2251. The rack gear 2251 is a member for guiding the vertical movement of the massage module 2170, and may include a plurality of valleys and a plurality of crests.

According to an embodiment of the present invention, the rack gears 2251 may be disposed at both sides of the upper frame 2250 to face each other, and the massage module 2170 may move along the rack gears 2251.

In FIG. 2, the rack gear 2251 is formed in a vertical direction, but is not limited thereto. The rack gear2251 may include a rack gear of a back-and-forth direction and a rack gear of a vertical direction. In the present invention, the back-and-forth direction refers to a direction heading from the massage module 2170 toward the user or from the user to the massage module 2170, and may also be referred to as a Z-axis direction.

The massage module 2170 may include a gear engaged with the rack gear 2251. More specifically, the massage module 2170 may include gears respectively engaged with the rack gear of the back-and-forth direction and the rack gear of the vertical direction. The gears are rotated by the actuator disposed in the massage module 2170, so that the massage module 2170 may move forward, backward, upward, or downward.

As the massage module 2170 moves forward, the strength of massage may be increased. Moreover, as the massage module 2170 moves backward, the strength of massage may be decreased.

The rack gear 2251 may be formed of a metal material or a plastic material. For example, the rack gear 2251 may be made of iron, steel, alloy, reinforced plastic, melamine resin, phenol resin, or the like, but is not limited thereto.

The upper frame 2250 may be formed in various shapes. For example, the upper frame 2250 may be one selected among an S frame, an L frame, an S&L frame, and a double S&L frame according to the shape of the upper frame, but is not limited thereto.

The S frame refers to a frame in which at least a portion of the upper frame 2250 has a curved shape like "S". The L frame refers to a frame in which at least a portion of the upper frame 2250 is bent like "L", the S&L frame refers to a frame having both of the curved shape like "S" and the bent shape like "L", and the double S&L frame refers to a frame having the curved shape like "L" and the curved shape like "S" formed at two portions.

The base frame 2210 supports the upper frame 2250 of the main frame 2110 and gets in contact with the ground. The base frame 2210 may include a base upper frame 2211 and a base lower frame 2212.

The base upper frame 2211 may support the upper frame 2250, and the base lower frame 2212 may get in contact with the ground. In addition, the base upper frame 2211 may be located in contact with the base lower frame 2212.

According to an embodiment of the present invention, the base upper frame 2211 may move along the base lower frame 2212. For example, the base upper frame 2211 may slide forward or backward along the base lower frame 2212. In this case, the upper frame 2250 may be connected to the base upper frame 2211 and move depending on the movement of the base upper frame 2211.

For example, in a case in which the base upper frame 2211 moves forward, the upper frame 2250 may also move forward together. In a case in which the base upper frame 2211 moves backward, the upper frame 2250 may also move backward together. Due to this, the sliding movement of the body massage unit 2100 may be allowed.

Specifically, in order to allow movement of the base upper frame 2211, a moving wheel may be provided beneath the base upper frame 2211. In addition, a guide member capable of guiding the moving wheel may be provided on an upper portion of the base lower frame 2212. The moving wheel provided beneath the base upper frame 2211 may move along the guide member provided on the base lower frame 2212, thereby allowing forward movement or backward movement of the base upper frame 2211.

According to another embodiment of the present invention, the massage apparatus 100 may not provide a sliding function, and in this case, the base frame 2210 may not be separated into upper and lower frames.

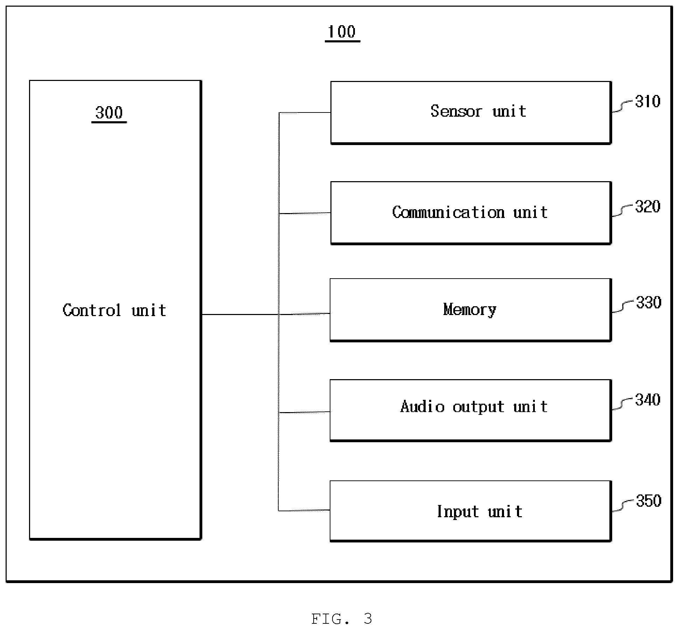

FIG. 3 is a view illustrating a massage apparatus according to an embodiment of the present invention.

The massage apparatus 100 may include at least one among a control unit 300, a sensor unit 310, a communication unit 320, a memory 330, an audio output unit 340, and an input unit 350. The control unit 300 may include at least one processor and a memory. The at least one processor may perform instructions stored in the memory.

The control unit 300 may control the operation of the massage apparatus 100. The control unit 300 may include one processor or may include a plurality of processors. In a case in which the control unit 300 includes a plurality of processors, at least a portion of the plurality of processors may be physically spaced apart from each other. In addition, the massage apparatus 100 is not limited thereto and may be implemented in various ways.

According to an embodiment of the present invention, the control unit 300 may control the operation of the massage apparatus 100. For example, the massage apparatus 100 may include a plurality of actuators. The massage apparatus 100 may control the operation of the massage apparatus 100 by controlling the operation of the plurality of actuators. For example, the massage apparatus 100 may include at least one among a driving unit for moving an armrest frame support unit, an actuator for moving the massage module 2170, at least one actuator included in the massage module, a back angle actuator, a leg angle actuator, a foot massage actuator, a leg length adjusting actuator, and a sliding actuator, and the control unit 300 may control the operation of the massage apparatus 100 by controlling them.

The massage module moving actuator is an actuator that enables vertical movement of the massage module 2170, and the massage module 2170 may move along the rack gear by the operation of the massage module moving actuator.

The back angle actuator is an actuator that adjusts an angle of a portion where the back of the user gets in contact with the massage apparatus 100, and the back angle of the massage apparatus 100 may be adjusted by the operation of the back angle actuator.

The leg angle actuator is an actuator for adjusting an angle of the leg massage unit 2300 of the massage apparatus 100. An angle between the leg massage unit 2300 and the body massage unit 2100 may be adjusted by the operation of the leg angle actuator.

The foot massage actuator is an actuator that operates the foot massage module included in the leg massage unit 2300. The massage apparatus 100 may provide foot massage to the user by using the foot massage actuator.

The massage module 2170 may include at least one actuator, and the control unit 300 may operate at least one actuator to provide various massage motions. For example, the control unit 300 may operate at least one actuator included in the massage module 2170 to provide a tapping massage, a kneading massage, and the like, and may provide various massage operations without being limited thereto.

The leg length adjusting actuator represents an actuator that adjusts the length of the leg massage unit 2300. For example, the control unit 300 may adjust the length of the leg massage unit 2300 to be suitable for the user by using the leg length adjusting actuator, and as a result, the user may receive massage suitable for the user's body shape.

The sliding actuator enables a sliding motion of the massage apparatus 100. For example, a horizontal base upper frame may move forward or backward by the operation of the sliding actuator, and as a result, the upper frame connected to the horizontal base upper frame may also move forward or backward.

The memory 330 may be included in the control unit 300 or may be located outside the control unit 300. The memory 330 may store various pieces of information related to the massage apparatus 100. For example, the memory 330 may include massage control information and personal authentication information, but is not limited thereto.

The memory 330 may be implemented through a non-volatile storage medium capable of continuously storing arbitrary data. For example, the memory 330 may include not only a disk, an optical disk, and a magneto-optical storage device, but also a storage device based on a flash memory and/or a battery-backup memory, but is not limited thereto.

The memory 330 may be a primary storage device, to which a processor directly accesses, like a random access memory (RAM), such as a dynamic random access memory (DRAM), a static random access memory (SR_AM), or the like. The memory 330 may mean a volatile storage device in which stored information is instantaneously erased when power is turned off, but is not limited thereto. The memory 330 may be operated by the control unit 300.

The massage apparatus 100 may include a sensor unit 310. The sensor unit 310 may acquire varied information using at least one sensor. The sensor unit 310 may be a sensor using a measurement means such as pressure, potential, and optics. For example, the sensor may include a pressure sensor, an infrared sensor, an LED sensor, a touch sensor, and the like, but is not limited thereto.

In addition, the sensor unit 310 may include a biometric information acquisition sensor. The biometric information acquisition sensor may acquire fingerprint information, face information, voice information, iris information, weight information, electrocardiogram information, body composition information, and the like, but is not limited thereto, and may include various pieces of biometric information. For instance, the biometric information acquisition sensor may include at least one among a first bio-signal measurement unit, a second bio-signal measurement unit, a third bio-signal measurement unit, and a fourth bio-signal measurement unit, which will be described later.

According to another embodiment of the present invention, the massage apparatus 100 may sense a contact area and/or a contact position with the user through sensors. Furthermore, the massage apparatus 100 may provide a customized massage based on information acquired through sensors. In addition, the massage apparatus 100 may include a communication unit. The communication unit of the massage apparatus 100 may receive a signal from an external device. The control unit 300 may process the received signal to obtain a result signal. The communication unit may output the result signal to the external device.

The communication unit 320 may communicate with a module inside the massage apparatus 100, an external massage apparatus, and/or a user terminal through any type of network. The communication unit may include a wired/wireless access module for network access. The wireless access technology may include, for example, wireless LAN (WLAN) (Wi-Fi), wireless broadband (Wibro), world interoperability for microwave access (Wimax), high speed efficient downlink alliance (HSDPA), and the like. The wired access technology may include, for example, digital subscriber line (XDSL), fibers shop to the home (FTTH), power line communication (PLC), and the like. In addition, the network connection unit may include a short-range communication module to transmit and receive data to and from any device/terminal located at a short distance. For example, the short-range communication technology may include Bluetooth, radio frequency identification (RFID), infrared data security association (IrDA), ultra-wideband (UWB), ZigBee, and the like, but is not limited thereto.

Additionally, the massage apparatus 100 may further include an input unit 350 and/or an output unit. The massage apparatus 100 may receive an input from the user by using the input unit 350. In addition, the massage apparatus 100 may output the processed result of the control unit 300 to the output unit.

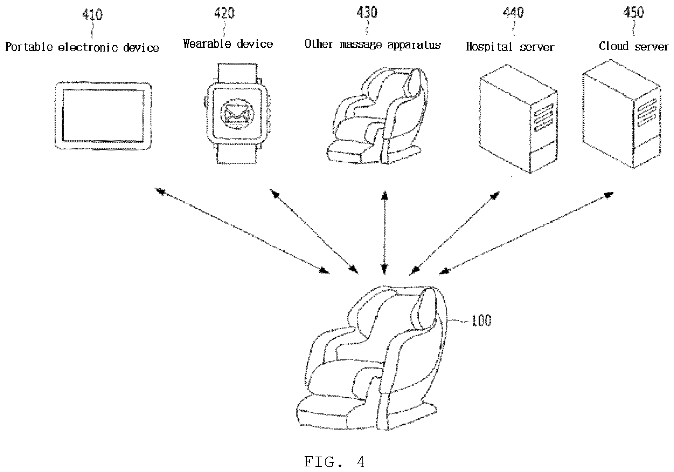

Specifically, the input unit 350 may receive a command related to the operation control of the massage apparatus 100 from the user, and the input unit 350 may be implemented in various forms. For example, the input unit 350 may be provided in the body massage unit 2100, or may be provided in the leg massage unit 2300, but is not limited thereto. Moreover, the input unit 350 may include a user input unit 2180, the massage apparatus control device 2200 of FIG. 1, or various external devices to be described referring to FIG. 4.

The massage apparatus 100 may acquire various commands from the user through the input unit 350. For example, the massage apparatus 100 may receive commands related to section of a massage module, selection of a massage type, selection of massage intensity, selection of massage time, selection of a massage area, selection of a position and an operation of the body massage unit 2100, selection of ON and OFF of power of the massage apparatus 100, selection of whether to operate a thermal function, selection related to sound reproduction, and the like, but is not limited thereto.

The massage apparatus 100 may provide an interface for selecting a massage mode. For example, the input unit 350 or the output unit may include a massage apparatus control device 2200. A medical massage list of various modes related to body improvement may be listed through the massage apparatus control device 2200.

The medical massage mode may include at least one among a concentration mode, a meditation mode, a recovery mode, a stretching mode, a sleep mode, a vital mode, a golf mode, a hip-up mode, an examinee mode, a gravity-free mode, and a growth mode.

According to another embodiment of the present invention, the input unit 350 may include buttons of a hot key type and/or selection buttons for selection, cancellation and input, according to preset user setting functions or preset self-setting functions.

The input unit 350 may be implemented as a keypad, a dome switch, a (static pressure type/capacitive) touch pad, a jog wheel, a jog switch, or the like, but is not limited thereto. Furthermore, the input unit 350 may obtain commands through the utterance of the user based on the voice recognition technology.

According to an embodiment of the present invention, the output unit may include a display for displaying an operation situation of the massage apparatus 100 or a current state of the user. In this case, the display may include at least one among a liquid crystal display (LCD), a thin film transistor-liquid crystal display (TFT-LCD), an organic light-emitting diode (OLED) display, a flexible display, and a three-dimensional (3D) display, but is not limited thereto.

The output unit may include an audio output unit 340. The audio output unit 340 of FIG. 3 may include the audio output module 2160 of FIG. 1. The audio output unit 340 may provide a user with an audio output of any form. For example, the audio output unit 340 may output a sound source and/or a binaural beat optimized for a massage pattern provided by the massage apparatus 100 to the user, thereby providing brain stimulation to the user. The audio output unit 340 may output a sound signal received through a network (not illustrated) or stored in an internal/external storage medium (not illustrated). For example, the audio output unit 340 may output a sound source according to the control of the user terminal through a network connection (e.g., Bluetooth connection, etc.) with the user terminal. Additionally, the audio output unit 340 may output a sound signal generated in relation to the operation of the massage apparatus 100.

It will be appreciated by those skilled in the art that the present invention may be implemented in combination with other program modules and/or through a combination of hardware and software. For example, the present invention may be implemented by a computer-readable medium.

Any of media accessible by a computer may be a computer-readable medium. Such a computer-readable medium may include a volatile medium, a non-volatile medium, a transitory medium, a non-transitory medium, a movable medium, and an unmovable medium. For example, the computer-readable medium may include a computer-readable storage medium and a computer-readable transmission medium.

The computer-readable storage medium includes a volatile medium, a non-volatile medium, a transitory medium, a non-transitory medium, a movable medium, and an unmovable medium implemented by computer-readable commands, a data structure, a program module, or a method or technology for storing information like data. The computer-readable storage medium includes a memory technology, such as a RAM, a ROM, an EEPROM, a flash memory or the like, a magnetic storage device, such as a CD-ROM, a digital video disk (DVD) or the like, a magnetic storage device, such as a magnetic cassette, a magnetic tape, a magnetic disk storage or the like that can be accessed by a computer and that can be used to store desired information, but is not limited thereto.

FIG. 4 is a view illustrating an external device capable of communicating with the massage apparatus 100 according to an embodiment of the present invention.

The massage apparatus 100 may communicate with an external device in a wired or wireless manner to transmit and receive various data.

The external device may include a portable electronic device 410, such as an AI speaker, a tablet, or a smartphone. The portable electronic device 410 may be only for the massage apparatus 100 or may be a general-purpose portable electronic device. In addition, the external device may include a wearable device 420 such as a smart watch or a smart band. The external device may not include the massage apparatus 100 currently used by the user but other massage apparatus 430. The external device may include a hospital server 440. The external device may include a personal health record (PHR) server. Furthermore, the external device may include a cloud server 450. The external device may include a medical measurement device, such as an electronic scale, a blood glucose monitoring device, or a blood pressure meter.

Although some examples of the external device are described in the present invention, it should be understood that all kinds of devices capable of communicating with the massage apparatus 100 and sending and receiving information with the massage apparatus 100 in a wired or wireless manner may be included in an external device.

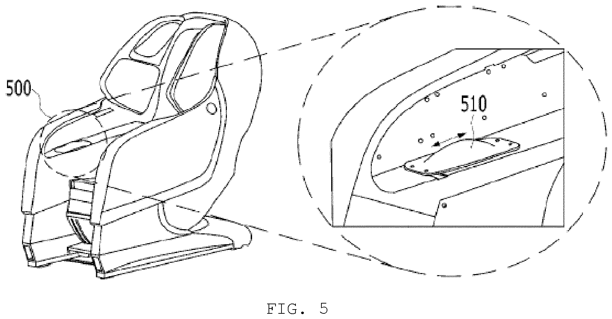

FIG. 5 is a view illustrating a first bio-signal measurement unit according to an embodiment of the present invention.

The massage apparatus 100 may include a first bio-signal measurement unit 510. The first bio-signal measurement unit 510 may be included in the sensor unit 310 of FIG. 3. The first bio-signal measurement unit 510 may be formed on an arm massage unit 500. The first bio-signal measurement unit 510 may be located at an area where the user's palm is placed. Moreover, the first bio-signal measurement unit 510 may include at least one electrode. The user's hand may be naturally positioned on the first bio-signal measurement unit 510 while receiving massage. The first bio-signal measurement unit 510 may measure the user's bio-signal using at least one electrode. The control unit 300 may obtain information on the user's physical condition based on the measured bio-signal. The information on the user's physical condition acquired by the first bio-signal measurement unit 510 may include body composition or electrocardiogram.

Body composition refers to information about body constituent parts. For example, body composition may include information about intracellular water, extracellular water, body water, protein, mineral, body fat, muscle mass, body fat mass, skeletal muscle mass, body fat percentage, BMI, muscle mass by region, body water by region, anasarca, edema by region, body cell mass, bone mineral content, percentage of abdominal fat, cross-sectional area of visceral fat, and basic metabolic rate, but is not limited thereto.

In a case in which the massage apparatus 100 sends a weak alternating current to the human body through the bio-signal measurement unit, the current flows along the highly conductive body water, and the width of a passage through which electricity flows is determined according to the amount of body water. It appears as a measured value called impedance, and the massage apparatus can calculate body composition by using the measured impedance. In this case, the massage apparatus 100 may calculate body composition by using a 2-electrode 4-touch electrode method using two electrodes, and may calculate body composition by using a 4-electrode 8-touch electrode method using four electrodes.

The electrocardiogram may be a record of the electrical activity of the heart. The electrocardiogram may be recorded by electrodes attached to the skin and by devices outside the body. The electrocardiogram may be recorded with an ammeter by inducing an action current generated from the myocardia according to the heartbeat to two appropriate electrodes on the body surface. The electrocardiogram is used not only to measure the rate and regularity of heartbeats, but also to determine the size and location of the heart and whether there is any damage to the heart.

The first bio-signal measurement units 510 may be respectively disposed on right and left arm massage units. The first bio-signal measurement unit of the left side may include two electrodes. Additionally, the first bio-signal measurement unit of the right side may include two electrodes. The first bio-signal measurement unit 510 may have a total of four electrodes including the left and right sides. The control unit 300 may measure a bio-signal of the user by using at least two electrodes among the electrodes included in the first bio-signal measurement unit located on the user's hand and the second bio-signal measurement unit located on the user's foot.

The first bio-signal measurement unit 510 can be moved back and forth based on the location of the user's hand. For example, the control unit 300 may receive a signal from a sensor for measuring the position of the user's hand. In order to measure the user's hand, a camera, an optical sensor, or an ultrasonic sensor may be used, but is not limited thereto, and various sensors capable of recognizing the position of the user's hand may be used. The control unit 300 may automatically move the first bio-signal measurement unit 510 based on the position of the user's hand. The control unit 300 may move the first bio-signal measurement unit 510 forward and backward. Here, the terms of forward or backward mean the forward or backward direction from the user when the user sits on the massage apparatus 100. The user may comfortably put the hand on the first bio-signal measurement unit 510.

However, the present invention is not limited to the first bio-signal measurement unit 510 moving automatically. The user may manually move the first bio-signal measurement unit 510. The user may pull or push the first bio-signal measurement unit 510 to move it. The first bio-signal measurement unit 510 may move forward and backward by the user's force.

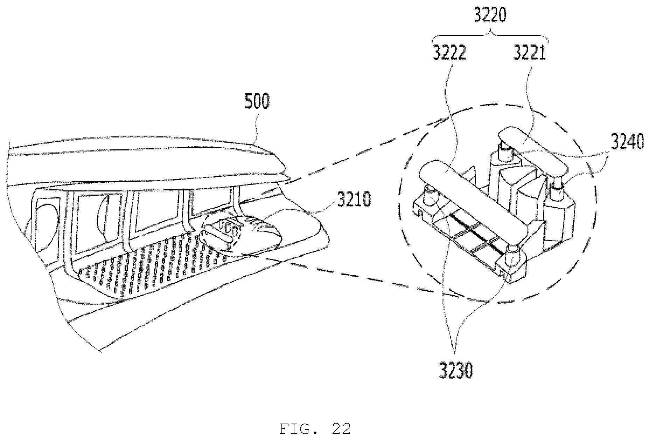

FIG. 22 is a view for depicting a third bio-signal measurement unit according to an embodiment of the present invention.

The massage apparatus 100 may include a third bio-signal measurement unit 3210. FIG. 22 illustrates the third bio-signal measurement unit 3210 disposed inside the massage apparatus 100. The third bio-signal measurement unit 3210 may replace the first bio-signal measurement unit 510.

The third bio-signal measurement unit 3210 may be at least partially hidden by a cover sheet. However, FIG. 22 illustrates a state in which the cover sheet is removed for convenience of description. The cover sheet may be a type of housing that covers a portion of the arm massage unit 500 so that the arm massage unit 500 looks clean. The cover sheet may be made of polyurethane (PU), polystyrene, or a sponge material.

The third bio-signal measurement unit 3210 is disposed (or may be formed or prepared) on the arm massage unit 500. The third bio-signal measurement unit 3210 may be located at an area where the user's palm is placed. Furthermore, the third bio-signal measurement unit 3210 may include at least one electrode. The user's palm may be naturally positioned on the third bio-signal measurement unit 3210 while the user gets massage. The third bio-signal measurement unit 3210 may measure the user's bio-signal by using at least one electrode. The control unit 300 may obtain information on the user's physical condition based on the measured bio-signal. The information on the user's physical condition acquired by the third bio-signal measurement unit 3210 may include body composition or electrocardiogram.

The massage apparatus 100 may include two third bio-signal measurement units 3210. The third bio-signal measurement units 3210 may be respectively disposed on the left and right arm massage units. The third bio-signal measurement unit of the left side may include two electrodes. Additionally, the third bio-signal measurement unit of the right side may include two electrodes. The third bio-signal measurement units 3210 may have a total of four electrodes including the left and right sides. The control unit 300 may measure the user's bio-signal by using at least two among the electrodes included in the third bio-signal measurement unit 3210 located on the user's hand, the second bio-signal measurement unit 610 located on the user's foot, or the fourth bio-signal measurement unit 3310.

The third bio-signal measurement unit 3210 may not be movable forward and backward unlike the first bio-signal measurement unit 510. The massage apparatus 100 may include any one among the first bio-signal measurement unit 510 and the third bio-signal measurement unit 3210. Since the third bio-signal measurement unit 3210 is located where the user can comfortably place the hand, the user can comfortably put the hand on the third bio-signal measurement unit 3210.

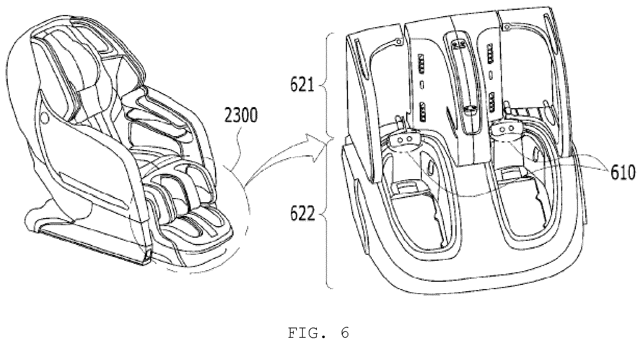

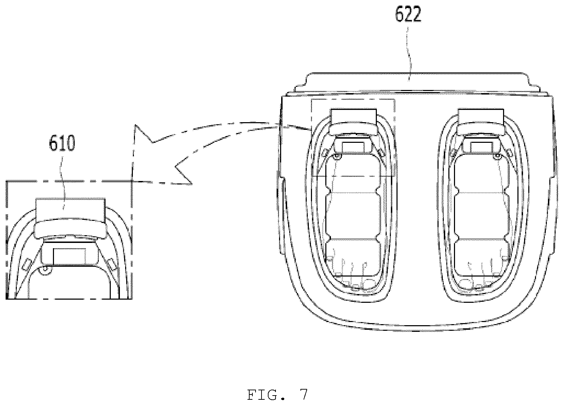

FIG. 6 is a view for depicting a second bio-signal measurement unit according to an embodiment of the present invention. Moreover, FIG. 7 is a view for depicting a method for a user to measure body composition through a second bio-signal measurement unit according to an embodiment of the present invention.

The massage apparatus 100 may include a second bio-signal measurement unit 610. The second bio-signal measurement unit 610 may be included in the sensor unit 310 of FIG. 3. The second bio-signal measurement unit 610 may be arranged on the foot massage unit 622. As described above, the foot massage unit 622 may be included in the leg massage unit 2300. The second bio-signal measurement unit 610 is located on the back of the user's ankle and may include at least one electrode.

The second bio-signal measurement unit 610 may be disposed on the foot massage unit 622. The massage apparatus 100 may measure body composition using a bipolar electrode method by utilizing the first bio-signal measurement unit 510 or the second bio-signal measurement unit 610. The massage apparatus 100 may measure body composition using a quadrupolar electrode method by utilizing both of the first bio-signal measurement unit 510 and the second bio-signal measurement unit 610, but is not limited thereto, and may measure body composition using a multi-polar electrode method.

In addition, according to another embodiment of the present invention, the massage apparatus 100 may measure body composition using the bipolar electrode method by individually utilizing the first bio-signal measurement unit 510 and the second bio-signal measurement unit 610, and may obtain the user's body composition by selecting a highly reliable measurement result through a reliability test.

Furthermore, the massage apparatus 100 may measure an electrocardiogram by utilizing the first bio-signal measurement unit 510 or the second bio-signal measurement unit 610.

The second bio-signal measurement unit 610 may be arranged on at least a portion of the leg massage unit 2300 so as to get in contact with at least a portion of the leg of the user when the user sits on the massage apparatus 100.

For instance, the leg massage unit 2300 is divided into a calf massage unit 621 massaging the user's calves and a foot massage unit 622 massaging the user's feet, and the second bio-signal measurement unit 610 may be located between the calf massage unit 621 and the foot massage unit 622. In addition, the second bio-signal measurement unit 610 may be provided on at least a portion of the foot massage unit 622, or may be located on at least a portion of the calf massage unit 621.

The second bio-signal measurement unit 610 may come into contact with a portion between the user's calf and foot in a case in which the user sits on the massage apparatus 100. For example, in a case in which the user sits on the massage apparatus 100, the second bio-signal measurement unit 610 may come into contact with the back of the user's ankle.

The second bio-signal measurement units 610 may be respectively located on the left and right leg massage units 2300. The second bio-signal measurement unit of the left side may include two electrodes. Additionally, the second bio-signal measurement unit of the right side may include two electrodes. The second bio-signal measurement unit may have a total of four electrodes including the left and right sides. The control unit 300 may measure the user's bio-signal by using at least one among the electrodes included in the first bio-signal measurement unit 510 or the third bio-signal measurement unit 3210 located on the user's hand and at least one among the electrodes included in the second bio-signal measurement unit 610 located on the user's foot.

The second bio-signal measurement unit 610 is movable forward and backward. More specifically, at least one electrode included in the second bio-signal measurement unit 610 is movable forward and backward. The user may move the second bio-signal measurement unit 610. The second bio-signal measurement unit 610 may be usually located in front by a spring. However, in a case in which the user sits on the massage apparatus 100 and puts the legs or feet on the second bio-signal measurement unit 610, the second bio-signal measurement unit 610 may be moved backward by the user's legs or feet. Here, the terms of *'*front*'* and `back' may mean the front and the back of the foot of the user. For instance, the 'front' may mean the user's toes, and the `back' may mean the user's heel. In addition, while the user is sitting on the massage apparatus 100, the front and back may mean the front and back of the user. That is, the front may mean a toe direction and the back may mean a heel direction. Additionally, in a state in which the user lies down with his/her legs stretched on the massage apparatus 100, the front may mean the sky direction, and the back may mean the ground direction. As described above, since the second bio-signal measurement unit 610 or the electrode may move forward and backward, at least one electrode may get in close contact with the user's foot or leg to measure a bio-signal.

The second bio-signal measurement unit 610 may be used in a case in which an angle 3351 that the leg massage unit 2300 is perpendicular to the ground surface is greater than or equal to a predetermined angle. The massage apparatus 100 may incline the leg massage unit 2300. More specifically, the basic state of the massage apparatus 100 may be a state in which the leg massage unit 2300 is substantially perpendicular to the ground. The massage apparatus 100 may tilt the leg massage unit 2300 to make the user lie down. As the leg massage unit 2300 is inclined, the user's knees and legs may be stretched. In addition, as the leg massage unit 2300 is inclined, the angle between the vertical line of the ground and the leg massage unit 2300 may gradually increase. In a case in which the angle between the vertical line of the ground and the leg massage unit 2300 is greater than or equal to a predetermined angle, the massage apparatus 100 may measure the bio-signal by using the second bio-signal measurement unit 610. The user's legs may be forced down by gravity. Therefore, in a case in which the angle between the vertical line of the ground and the leg massage unit 2300 is greater than or equal to the predetermined angle, the user's heel gets in close contact with the second bio-signal measurement unit 610, and the massage apparatus 100 may accurately measure the user's bio-signal. Additionally, in a case in which the angle between the vertical line of the ground and the leg massage unit 2300 is less than the predetermined angle, the massage apparatus 100 may measure the bio-signal by using the fourth bio-signal measurement unit 3310. The fourth bio-signal measurement unit 3310 will be described in more detail later.

The control unit 300 may obtain information on the user's physical condition based on the bio-signal acquired from at least two bio-signal measurement units among the first bio-signal measurement units 510 to the fourth bio-signal measurement unit 3310. The bio-signal may be a value measured by applying current or voltage to the user's body. For example, the bio-signal may include at least one among impedance, a current value, or a voltage value obtained from at least two bio-signal measurement units among the first bio-signal measurement units 510 to the fourth bio-signal measurement unit 3310. The control unit 300 may obtain information on the biological state based on the bio-signal. Information on the physical condition may include body composition or electrocardiogram.

Biometric information for obtaining body composition or electrocardiogram included in information on the physical condition may be duplicated. The control unit 300 may acquire both body composition information and electrocardiogram information by measuring biometric information once without separately measuring biometric information to obtain body composition information and electrocardiogram information, but is not limited thereto. The control unit 300 may separately measure a bio-signal for acquiring body composition information and a bio-signal for acquiring electrocardiogram information.

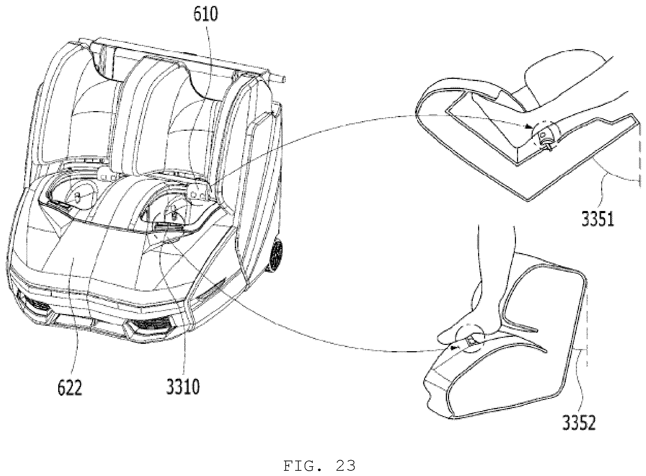

FIG. 23 is a view for depicting a second bio-signal measurement unit and a fourth bio-signal measurement unit according to an embodiment of the present invention.

As illustrated in FIGS. 6 and 7, the massage apparatus 100 may include the second bio-signal measurement unit 610, but is not limited thereto. Referring to FIG. 23, the massage apparatus 100 may include a fourth bio-signal measurement unit 3310 instead of the second bio-signal measurement unit 610. Moreover, the massage apparatus 100 may include both of the second bio-signal measurement unit 610 and the fourth bio-signal measurement unit 3310.

The fourth bio-signal measurement unit 3310 may be included in the sensor unit 310 of FIG. 3. The fourth bio-signal measurement unit 3310 may be formed on the foot massage unit 622. As described above, the foot massage unit 622 may be included in the leg massage unit 2300. The fourth bio-signal measurement unit 3310 may be formed on the upper surface of a housing of the foot massage unit 622. Additionally, the fourth bio-signal measurement unit 3310 may include at least one electrode. For instance, the fourth bio-signal measurement unit 3310 may include at least one electrode for measuring a fourth bio-signal.

The fourth bio-signal measurement unit 3310 may be disposed on the foot massage unit 622. The massage apparatus 100 may measure body composition using a bipolar electrode method or a quadrupolar electrode method by utilizing at least two among the first bio-signal measurement unit 510, the second bio-signal measurement unit 610, the third bio-signal measurement unit 3210, and the fourth bio-signal measurement unit 3310, but is not limited thereto. The massage apparatus 100 may measure body composition using a multipolar electrode method.

In addition, according to another embodiment of the present invention, the massage apparatus 100 may measure body composition using the bipolar electrode by selecting two among the first bio-signal measurement unit 510, the second bio-signal measurement unit 610, the third bio-signal measurement unit 3210, and the fourth bio-signal measurement unit 3310, and may obtain the user's body composition by selecting a highly reliable measurement result through a reliability test. The massage apparatus 100 may obtain body composition values by the at least two bio-signal measurement units selected among the first bio-signal measurement unit 510, the second bio-signal measurement unit 610, the third bio-signal measurement unit 3210, and the fourth bio-signal measurement unit 3310. More specifically, the massage apparatus 100 may obtain body composition values by selecting at least two bio-signal measurement units selected among the first bio-signal measurement unit 510, the second bio-signal measurement unit 610, the third bio-signal measurement unit 3210, and the fourth bio-signal measurement unit 3310. For instance, in a case in which the massage apparatus 100 obtains the body composition values by selecting two bio-signal measurement units, the combination of the bio-signal measurement units may the first bio-signal measurement unit 510 and the second bio-signal measurement unit 610, the first bio-signal measurement unit 510 and the third bio-signal measurement unit 3210, the first bio-signal measurement unit 510 and the fourth bio-signal measurement unit 3310, the second bio-signal measurement unit 610 and the third bio-signal measurement unit 3210, the second bio-signal measurement unit 610 and the fourth bio-signal measurement unit 3310, and the third bio-signal measurement unit 3210 and the fourth bio-signal measurement unit 3310. The massage apparatus 100 may obtain the body composition values with respect to each combination of the bio-signal measurement units.

The fourth bio-signal measurement unit 3310 may be used in a case in which an angle 3352 that the leg massage unit 2300 is perpendicular to the ground surface is less than a predetermined angle. The massage apparatus 100 may incline the leg massage unit 2300. More specifically, the basic state of the massage apparatus 100 may be a state in which the leg massage unit 2300 is substantially perpendicular to the ground. In this instance, the user's knees are in a bent state, and the user's fibulas are substantially perpendicular to the ground. The massage apparatus 100 may tilt the leg massage unit 2300 to make the user lie down. As the leg massage unit 2300 is inclined, the user's knees and legs may be stretched. In addition, as the leg massage unit 2300 is inclined, the angle between the vertical line of the ground and the leg massage unit 2300 may gradually increase. In a case in which the angle between the vertical line of the ground and the leg massage unit 2300 is greater than or equal to a predetermined angle, the massage apparatus 100 may measure the bio-signal by using the second bio-signal measurement unit 610. In a case in which the angle between the vertical line of the ground and the leg massage unit 2300 is less than the predetermined angle, the massage apparatus 100 may measure the bio-signal by using the fourth bio-signal measurement unit 3310. In the case in which the angle between the vertical line of the ground and the leg massage unit 2300 is less than the predetermined angle, the user's calves may be substantially perpendicular to the ground. In addition, since the user's legs are forced by gravity, the sole surface of the user may come into close contact with the fourth bio-signal measurement unit 3310, and the massage apparatus 100 may accurately measure the user's bio-signal.

The massage apparatus 100 may output a usable measuring unit among the second bio-signal measurement unit 610 and the fourth bio-signal measurement unit 3310 into a sound or an image according to the angle between the vertical line of the ground and the leg massage unit 2300. The user can measure the bio-signal by using any one among the second bio-signal measurement unit 610 and the fourth bio-signal measurement unit 3310 according to a guide output from the massage apparatus 100.

The massage apparatus 100 may obtain an average value of the body composition values with respect to the combination of the bio-signal measurement units. Moreover, the massage apparatus 100 may determine a value most similar to the average value of the body composition values with respect to the combination as a highly reliable measurement result. The similar value may be one among the body composition values by the two bio-signal measurement unit selected among the first bio-signal measurement unit 510, the second bio-signal measurement unit 610, the third bio-signal measurement unit 3210, and the fourth bio-signal measurement unit 3310, but is not limited thereto. The massage apparatus 100 may determine the average value of the body composition values with respect to the combination as a highly reliable measurement result.

In addition, the massage apparatus 100 may measure an electrocardiogram by utilizing at least one among the first bio-signal measurement unit 510, the second bio-signal measurement unit 610, the third bio-signal measurement unit 3210, and the fourth bio-signal measurement unit 3310.

The fourth bio-signal measurement unit 3310 is disposed on at least a portion of the leg massage unit 2300. In a case in which the user sits on the massage apparatus 100 and puts the legs on the fourth bio-signal measurement unit 3310, the sole of user may come into contact with the electrode of the fourth bio-signal measurement unit 3310.

The massage apparatus may include two fourth bio-signal measurement units 3310. The fourth bio-signal measurement units 3310 may be respectively located on the left leg massage unit 2300 and the right leg massage unit 2300. The fourth bio-signal measurement unit of the left side may include two electrodes. Furthermore, the fourth bio-signal measurement unit of the right side may include two electrodes. The fourth bio-signal measurement unit may have a total of four electrodes including the left and right sides. The control unit 300 may measure the user's bio-signal by using at least two among the electrodes included in the first bio-signal measurement unit 510, the second bio-signal measurement unit 610, the third bio-signal measurement unit 3210, and the fourth bio-signal measurement unit 3310.

The control unit 300 may obtain information the user's physical condition based on the bio-signal obtained from the first bio-signal measurement unit 510, the second bio-signal measurement unit 610, the third bio-signal measurement unit 3210, or the fourth bio-signal measurement unit 3310. For example, the control unit 300 may measure the user's bio-signal by using at least two among total four third bio-signal measurement electrodes 3220 and at least two among total four fourth bio-signal measurement electrodes, and obtains information on the user's physical condition based on the user's bio-signal.

The bio-signal may be a value measured by applying current or voltage to the user's body. For example, the bio-signal may include at least one among impedance, a current value and a voltage value obtained from the first bio-signal measurement unit 510, the second bio-signal measurement unit 610, the third bio-signal measurement unit 3210, or the fourth bio-signal measurement unit 3310. The control unit 300 may obtain information on the physical condition based on the bio-signal. Information on the physical condition may include body composition or electrocardiogram.

Biometric information for obtaining body composition or electrocardiogram included in information on the physical condition may be duplicated. The control unit 300 may acquire both body composition information and electrocardiogram information by measuring biometric information once without separately measuring biometric information to obtain body composition information and electrocardiogram information, but is not limited thereto. The control unit 300 may separately measure a bio-signal for acquiring body composition information and a bio-signal for acquiring electrocardiogram information.

Hereinafter, components included in the first bio-signal measurement unit 510 and the second bio-signal measurement unit 610 will be described in detail.

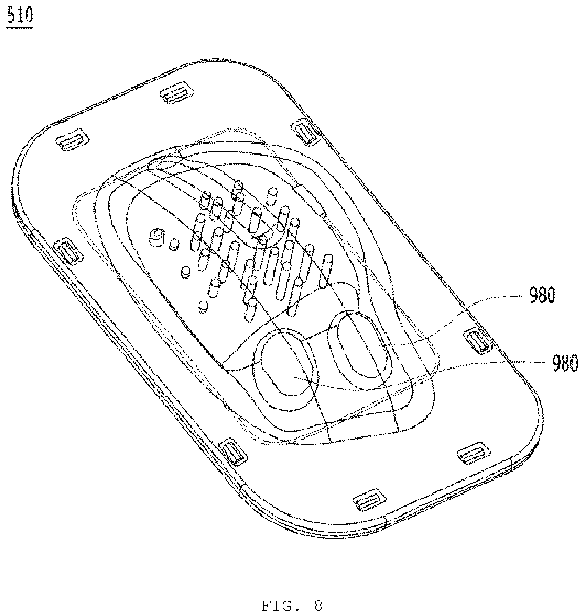

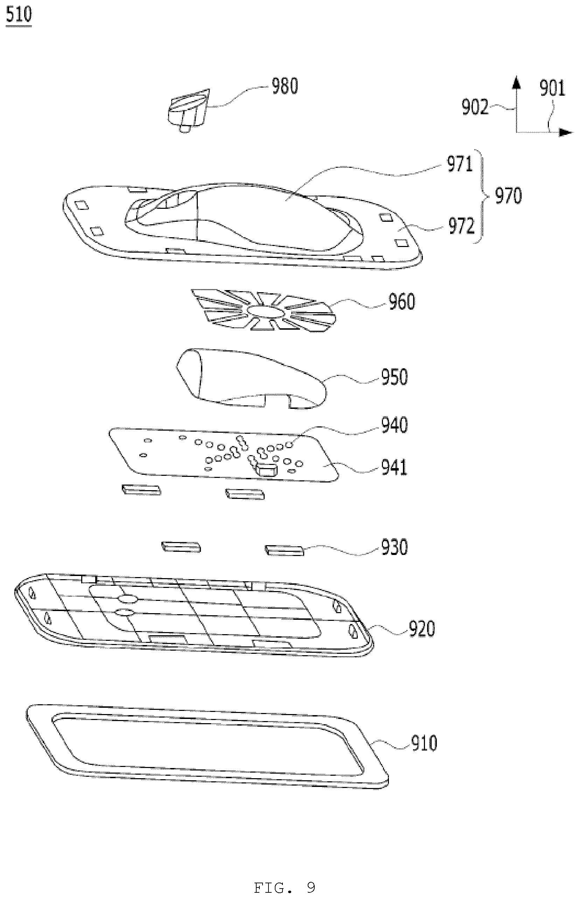

FIG. 8 is a perspective view of a first bio-signal measurement unit according to an embodiment of the present invention. FIG. 9 is a view for depicting components included in the first bio-signal measurement unit according to an embodiment of the present invention.

In FIG. 9, the word 'front' (901) may mean the forward direction when the user sits on the massage apparatus 100. In addition, the word 'upward' or 'above' (902) may mean the upward direction when the user sits on the massage apparatus 100.

The first bio-signal measurement unit 510 may include a plate 910. The plate 910 may be fixed on the housing of the arm massage unit 500. The plate 910 may be made of a metal material. The plate may have a shape elongated to the front (901) or to the back. The plate 910 may have a doughnut shape. That is, the plate 910 may have a hole.

The first bio-signal measurement unit 510 may include a lower cover 920. The lower cover 920 may be put on the plate. As described above, the plate 910 may be fixed to the housing of the arm massage unit. However, the lower cover 920 may not be fixed to the plate 910. The lower cover 920 may be slidable on the plate 910.

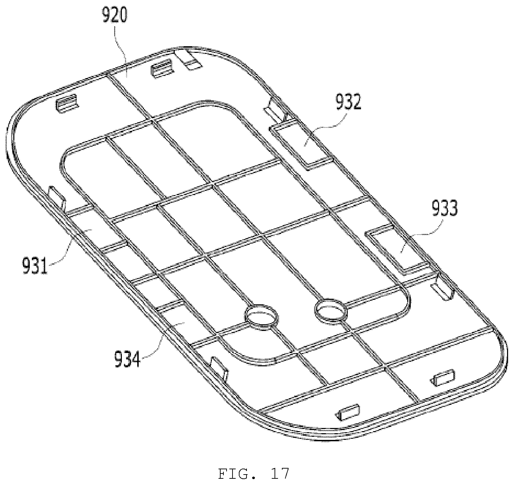

The first bio-signal measurement unit 510 may include at least two magnets 930. The at least two magnets 930 may be fixed on the lower cover 920. Referring to FIG. 17, the location of the magnets 930 according to an embodiment of the present invention will be described.

FIG. 17 is a view for depicting magnets according to an embodiment of the present invention.

Referring to FIG. 17, at least two magnets 930 may be disposed at both sides of the lower cover 920. The at least two magnets 930 may be fixed on the lower cover 920. The at least two magnets 930 may include a first magnet 931, a second magnet 932, a third magnet 933, and a fourth magnet 934. The first magnet 931 and the fourth magnet 934 may be located at the left edge of the lower cover 920. The second magnet 932 and the third magnet 933 may be located at the right edge of the lower cover 920.

Referring to FIG. 9, the at least two magnets 930 may face the plate 910. The at least two magnets 930 may not touch the plate 910 by the lower cover 920. However, the at least two magnets 930 and the plate 910 may attract each other by a magnetic force. One of the at least two magnets 930 may be located at the left edge of the lower cover 920, and the other one may be located at the right edge of the lower cover 920.

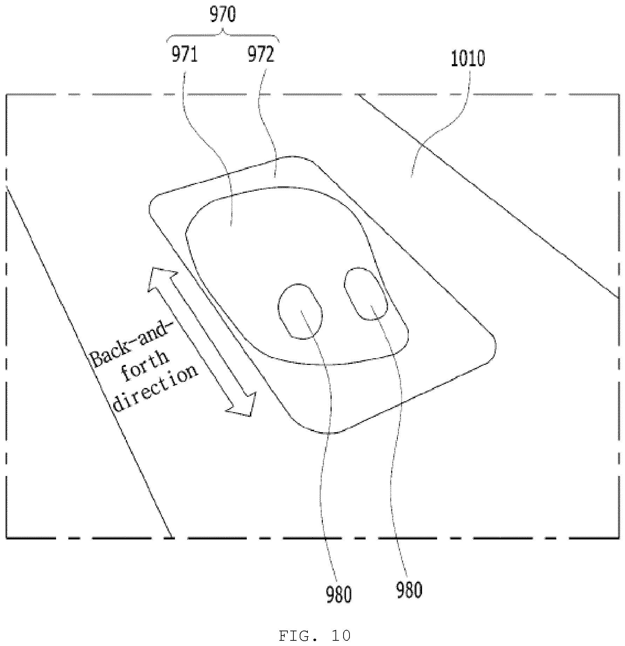

Due to the at least two magnets 930, the lower cover 920 may move forward (901) or backward on the plate 910. The movement of the first bio-signal measurement unit 510 will be described with reference to FIG. 10. Referring to FIG. 9, the first bio-signal measurement unit 510 may include an upper cover 970. The upper cover 970 may cover the lower cover 920 to protect various components on the lower cover 920. The upper cover 970 may be coupled with the lower cover 920. The upper cover 970 may include a palm support 971. The palm support 971 may have an upward convex shape so that the user can comfortably put the hand on the palm support.

The first bio-signal measurement unit 510 may include a first bio-signal measurement electrode 980. The first bio-signal measurement electrode 980 may have very low resistance. Additionally, the first bio-signal measurement electrode 980 may be plated with nickel or chrome. Furthermore, the first bio-signal measurement electrode 980 may be a silicon electrode. The silicon electrode may be an electrode using silicon and silver nanowire. The silver nanowire has excellent conductivity and the flexible nature. Since the silicon electrode is flexible, even if it touches the user's skin, the user does not feel cold and hard, but may feel comfortable. In addition, the silicon electrode is easy to have a desired shape.

The first bio-signal measurement electrode 980 may touch a portion of the palm of the user. The first bio-signal measurement electrode 980 may be inserted into a hole formed in the upper cover 970.

Two first bio-signal measurement electrodes 980 may be included in the first bio-signal measurement unit 510 of the left side. Moreover, two first bio-signal measurement electrodes 980 may be included in the first bio-signal measurement unit 510 of the right side. Therefore, the first bio-signal measurement units 510 of the left side and the right side may have a total of four first bio-signal measurement electrodes 980.

The control unit 300 may measure the user's bio-signal by using at least one among the first bio-signal measurement electrode 980 and the second bio-signal measurement electrode 610. In addition, the control unit 300 may obtain information on the physical condition based on the bio-signal. The first bio-signal measurement electrode 980 may touch the user. The first bio-signal measurement electrode 980 may be electrically connected to the control unit 300. The control unit 300 may obtain the bio-signal based on the signal from the bio-signal measurement electrode 980. Additionally, the control unit 300 may obtain information on the user's physical condition based on the bio-signal.

Referring to FIG. 22, the third bio-signal measurement unit 3210 may include the third bio-signal measurement electrode 3220.

The third bio-signal measurement electrode 3320 may be very lower in resistance. Furthermore, the third bio-signal measurement electrode 3320 may be plated with nickel or chrome. Furthermore, the third bio-signal measurement electrode 3320 may be a silicon electrode. The silicon electrode may be an electrode using silicon and silver nanowire. The silver nanowire has excellent conductivity and the flexible nature. Since the silicon electrode is flexible, even if it touches the user's skin, the user does not feel cold and hard, but may feel comfortable. In addition, the silicon electrode is easy to have a desired shape.

The third bio-signal measurement electrode 3320 may include a third-first bio-signal measurement electrode 3221 and a third-second bio-signal measurement electrode 3222. The third-first bio-signal measurement electrode 3221 and the third-second bio-signal measurement electrode 3222 may be elongated from the left to the right. Here, the left and the right may mean the left and the right when the user sits on the massage apparatus.

The third-first bio-signal measurement electrode 3221 may face upward to touch the user's hand. Furthermore, the third-first bio-signal measurement electrode 3221 may be fixed to the housing of the arm massage unit. In addition, the third-second bio-signal measurement electrode 3222 may be longer than the third-first bio-signal measurement electrode. The third-second bio-signal measurement electrode 3222 may be located at the back of the third-first bio-signal measurement electrode 3221. Here, the back may mean the back when the user sits on the massage apparatus 100. The third-second bio-signal measurement electrode 3222 may face upward to get in touch with the user's hand, and may be fixed to the housing of the arm massage unit.

Bottom surfaces of the third-first bio-signal measurement electrode 3221 and the third-second bio-signal measurement electrode 3222 may be coupled with two electrode supports extending upward and downward. Here, *'*downward*'* may mean a direction to face the ground, and `upward' may mean the opposite direction of the downward direction. The bottom surface of the third-first bio-signal measurement electrode 3221 may be coupled with two supports 3240. Furthermore, the bottom surface of the third-second bio-signal measurement electrode 3222 may be coupled with two supports 3230. The electrode supports 3230 and 3240 may be screw-coupled with the housing of the arm massage unit. Therefore, the third bio-signal measurement electrode 3220 may be fixed to the arm massage unit.

The third bio-signal measurement unit 3210 may also include an upper cover 970. The upper cover 970 may cover the lower cover 920 to protect various components and structures on the lower cover 920. The upper cover 970 may be coupled with the lower cover 920. The upper cover 970 may include a palm support 971. The palm support 971 may have an upward convex shape so that the user can put comfortably put the hand on the palm support. The lower cover 920 of the third bio-signal measurement unit 3210 may be fixed to the arm massage unit. Therefore, the third bio-signal measurement unit 3210 may not move forward and backward. The massage apparatus 100 may include only one among the first bio-signal measurement unit 510 and the third bio-signal measurement unit 3210.

The electrode supports 3230 and 3240 may be surrounded by filler. The upper surfaces of the third-first bio-signal measurement electrode 3221 and the third-second bio-signal measurement electrode 3222 may protrude from the filler. Therefore, the third bio-signal measurement electrode 3220 may touch a portion of the palm of the user. The filler may be made of polyurethane (PU), polystyrene, or a sponge material. The upper cover 970 may be located on the filler. The third bio-signal measurement electrode 3220 may be arranged through the hole of the upper cover 970, and the user's palm may touch the third bio-signal measurement electrode 3220.

The third bio-signal measurement unit of the left side may include two third bio-signal measurement electrodes. Moreover, the third bio-signal measurement unit of the right side may include two third bio-signal measurement electrodes. Therefore, the third bio-signal measurement units 3210 of the left side and the right side may include a total of four third bio-signal measurement electrodes 3220.

The control unit 300 may measure the user's bio-signal by using at least one among electrodes of the third bio-signal measurement electrode 3220, the second bio-signal measurement unit 610, and the fourth bio-signal measurement electrode 3410. Additionally, the control unit 300 may acquire information on the physical condition based on the bio-signal. The third bio-signal measurement electrode 3220 may get in contact with the user. The third bio-signal measurement electrode 3220 may be electrically connected with the control unit 300. The control unit 300 may acquire a bio-signal based on a signal from the third bio-signal measurement electrode 3220. In addition, the control unit 300 may acquire information on the user's physical condition based on the bio-signal.

Referring to FIG. 9, the first bio-signal measurement unit 510 or the third bio-signal measurement unit 3210 may include a plurality of LEDs 940 for treatment. The plurality of LEDs 940 for treatment may be fixed on the lower cover 920. The plurality of LEDs 940 for treatment may emit light based on a control signal of the control unit 300. The plurality of LEDs 940 for treatment may be located on an LED control PCB 941. The LED control PCB 941 may include a processor and a memory. The control unit 300 may transmit the control signal to the LED control PCB 941. The LED control PCB 941 may turn on or off at least a portion of the LEDs 940 for treatment based on the control signal.

The plurality of LEDs 940 for treatment may emit light of a wavelength good for articulation health of the user. For instance, the wavelength of light emitted from the plurality of LEDs 940 for treatment may be in a range of 0.5 um (micrometer) to 2.5 um. More specifically, the wavelength of light emitted from the plurality of LEDs 940 for treatment may be in a range of 800 nm (nanometer) to 1000 nm. In addition, the power density of light emitted from the LEDs 940 may be in a range of 2.0 to 3.0 mW/cm², and the energy density may be in a range of 11.00 to 12.00 J/cm².

Since the user's hand is positioned on the first bio-signal measurement unit 510 or the third bio-signal measurement unit 3210, the plurality of LEDs 940 for treatment may emit light toward the user's palm. The joints of the hand of the user may be recovered by the light of the plurality of LEDs 940 for treatment. More specifically, the light of the plurality of LEDs 940 for treatment may be directed toward the user's fingers. The user may apply light of the LEDs 940 to the fingers to promote joint health.

The plurality of LEDs 940 for treatment may be located below the upper cover 970. At least a portion of the upper cover 970 may be transparent so that the light of the plurality of LEDs 940 for treatment is transmitted to the user's hand. Alternatively, at least a portion of the upper cover 970 may be translucent. Alternatively, at least a portion of the upper cover 970 may include a hole so that the light of the LEDs 940 is transmitted to the user's hand. The upper cover 970 may be made of a material that transmits only specific wavelengths. For example, the upper cover 970 may be made of a material that transmits only the wavelength of light emitted from the LEDs 940.

The plurality of LEDs 940 for treatment may be arranged along five lines. Each line may be located below the fingers. Accordingly, the plurality of LEDs 940 for treatment may effectively apply light to the user's fingers. In addition, the plurality of LEDs 940 for treatment may be arranged concentrically. Therefore, the plurality of LEDs 940 for treatment may effectively apply light to the user s palm.

The first bio-signal measurement unit 510 or the third bio-signal measurement unit 3210 may further include a heating unit support 950. The heating unit support 950 may be fixed on the lower cover 920. The heating unit support 950 may be located below a heating unit to maintain the shape of the heating unit. The heating unit support 950 may have an upwardly convex shape. In a case in which the user naturally puts the hand on the first bio-signal measurement unit 510 or the third bio-signal measurement unit 3210, when viewed from the user's palm, the user's palm may be concave. Accordingly, the heating unit support 950 may be upwardly (902) convex to effectively transmit heat to the user's palm.

The heating unit support 950 may be arranged above (902) the plurality of LEDs 940 for treatment. The heating unit support 950 may be made of a transparent material not to hide light of the LEDs 940.

The first bio-signal measurement unit 510 or the third bio-signal measurement unit 3210 may further include a heating unit 960. The heating unit 960 may be fixed on the heating unit support 950. The heating unit 960 may be formed along the convex surface of the heating unit support 950. The heating unit 960 may generate heat based on a control signal of the control unit 300. The heating unit 960 may provide thermal fomentation to the user.

The heating unit 960 may be located above the plurality of LEDs. The heating unit 960 may have a shape not to hide the light of the LEDs 940. For instance, the heating unit 960 may include at least one slit. Due to the slit, light of the LEDs 940 is not hidden by the heating unit 960, and can be transmitted to the user's palm.

FIG. 10 is a view for depicting a first bio-signal measurement unit according to an embodiment of the present invention.

The arm massage unit may include a cover sheet 1010. The cover sheet 1010 may be a component provided to complete the design of the massage apparatus 100. The cover sheet 1010 may be made of leather, artificial leather, or polyurethane (PU). Moreover, the cover sheet 1010 may be provided to fix the first bio-signal measurement unit 510.

Referring to FIGS. 9 and 10, the upper cover 970 may include an upper cover fixing portion 972. The upper cover fixing portion 972 may be formed along the circumference of the palm support 971 of the upper cover 970 and may be flat. The upper cover fixing portion 972 may include at least one hole or hooking portion. The hanging portion may have a hook shape. The at least one hole or hooking portion of the upper cover fixing portion 972 is coupled to a hooking portion or hole of the lower cover 920 so that the upper cover 970 may be fixed to the lower cover 920.

The cover sheet 1010 may cover at least a portion of the upper cover fixing portion 972. The at least a portion of the upper cover fixing portion 972 may be positioned under the cover sheet 1010 of the arm massage unit to prevent the upper cover 970 from being separated from the cover sheet 1010 of the arm massage unit. That is, the cover sheet 1010 can prevent the first bio-signal measurement unit 510 from moving horizontally or vertically. The first bio-signal measurement unit 510 can move only in the back-and-forth direction.

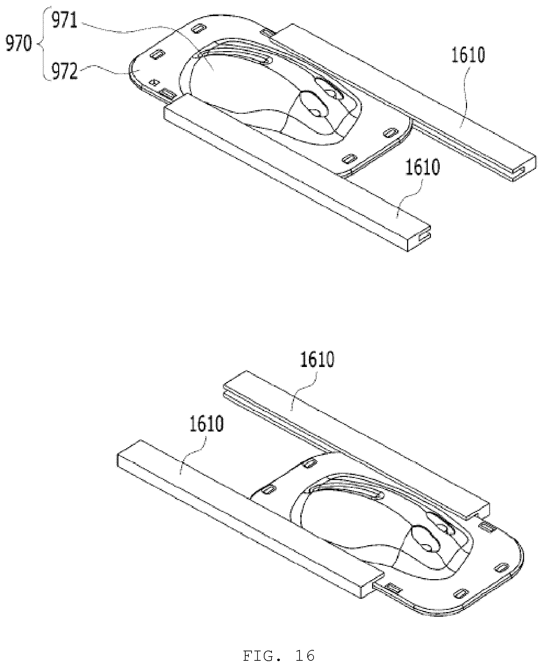

Although not illustrated in FIG. 9 or 10, guide rails may be formed on the plate 910 in the back-and-forth direction. The lower cover 920 may move back and forth along the guide rails. The guide rails may prevent the lower cover 920 from moving horizontally or vertically. Referring to FIG. 16, the plate 910 on which the guide rails are formed will be described.

FIG. 16 is a view for depicting a first bio-signal measurement unit according to an embodiment of the present invention.

The plate 910 according to an embodiment of the present invention may include guide rails 1610. The guide rails 1610 may be formed on the plate 910 in the back-and-forth direction. The guide rails 1610 may include grooves extending back and forth. The left side and the right side of the upper cover fixing portion 972 may be inserted into the grooves of the guide rails 1610. The first bio-signal measurement unit 510 may move back and forth along the guide rails 1610. The guide rails 1610 may prevent the upper cover 970 or the lower cover 920 from moving horizontally or vertically. That is, the guide rails 1610 may prevent the first bio-signal measurement unit 510 from moving horizontally or vertically.

According to an embodiment of the present invention, the first bio-signal measurement unit 510 may not include at least two magnets 930 since the first bio-signal measurement unit 510 can move back and forth by the guide rails 1610, but is not limited thereto. The first bio-signal measurement unit 510 may include magnets so that first bio-signal measurement unit 510 can firmly move on the plate 910 including the guide rails 1610.

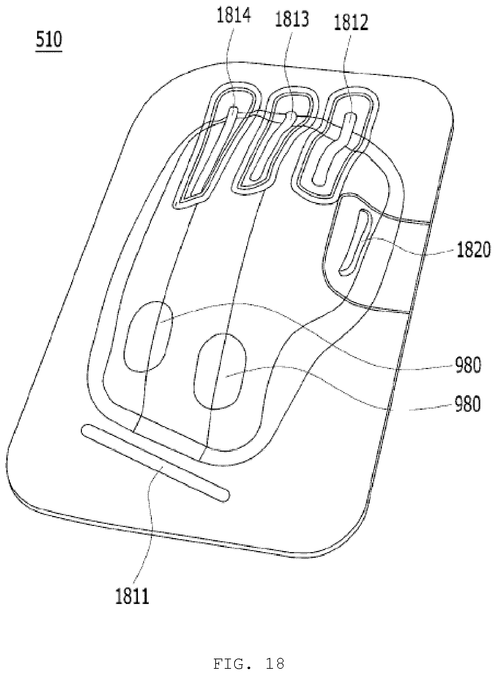

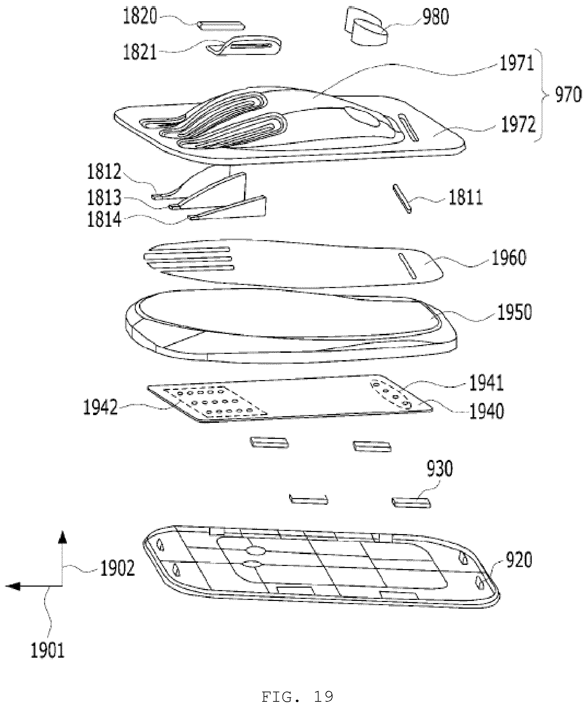

FIG. 18 is a perspective view of the first bio-signal measurement unit according to an embodiment of the present invention. FIG. 19 is a view for depicting components included in the first bio-signal measurement unit according to an embodiment of the present invention.

FIGS. 18 and 19 are views for depicting an embodiment different from the embodiment of FIGS. 8 and 9, but have components duplicating the components of FIGS. 8 and 9. In addition, the duplicated description thereof will be omitted. Therefore, undescribed contents with respect to FIGS. 18 and 19 can be understood with reference to FIGS. 8 and 9.

In FIG. 19, the word *'*front*'* (1901) may mean a direction that the user is looking at when the user sits on the massage apparatus 100. In addition, the word *'*upward*'* or *'*above*'* (1902) may mean the upward direction when the user sits on the massage apparatus 100.

Referring to FIGS. 18 and 19, the first bio-signal measurement unit 510 may include a function button 1820. The function button 1820 may be included in a user input unit 2180. The first bio-signal measurement unit 510 may include at least one function button 1820.

The function button 1820 may receive an input for a predetermined function. For example, the predetermined function may be a reclining on/off function or a massage mode start/stop function. However, the function button 1820 may not receive only an input for the predetermined function. The function of the function button 1820 may be set/changed by the control unit 300. The control unit 300 may change the function of the function button 1820 based on a user's input.

The first bio-signal measurement unit of the left side and the first bio-signal measurement unit of the right side may respectively include function buttons 1820. The function buttons 1820 included in the first bio-signal measurement unit of the left side and the first bio-signal measurement unit of the right side may perform different functions.

Referring to FIG. 19, the first bio-signal measurement unit 510 may include a function button guide 1821. The function button guide 1821 may be a structure for guiding the movement of the function button 1820.

The function button guide 1821 may be injection-molded separately from the upper cover 970. Additionally, the function button guide 1821 may be coupled to the upper cover 970, but is not limited thereto, and may be formed integrally with the upper cover 970.

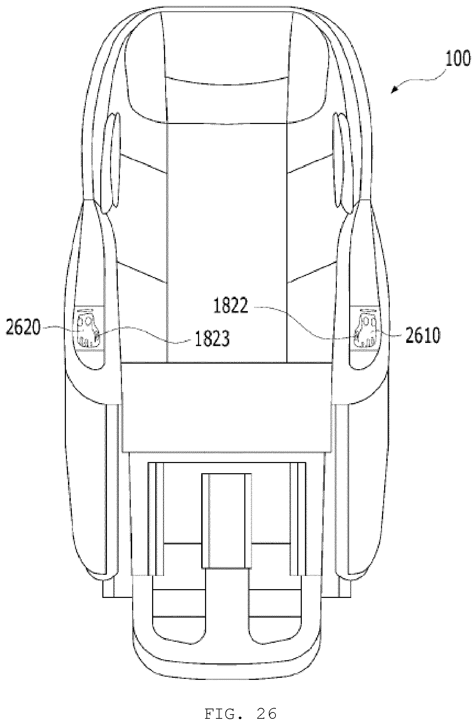

FIG. 26 is a view for depicting a function of a function button according to an embodiment of the present invention.

Referring to FIG. 26, the massage apparatus 100 may include a first bio-signal measurement unit 2610 of the left side arranged on a left armrest unit and a first bio-signal measurement unit 2620 of the right side arranged on a right armrest unit. The first bio-signal measurement unit 2610 of the left side may include a first function button 1822. Furthermore, the first bio-signal measurement unit 2620 of the right side may include a second function button 1823. The user can control the massage apparatus 100 by using the function buttons 1822 and 1823 arranged at the right and left sides even in a state in which both hands of the user are accommodated in the arm massage unit 500 to get a massage. That is, the user can easily manipulate various functions of the massage apparatus 100 even in a situation that the user is hard to move since the user's arms are grasped in the expanded arm airbags of the arm massage unit 500.

The massage apparatus 100 is configured for the user to set different functions of the function buttons 1822 and 1823 according to the current operation state of the massage apparatus 100. For instance, in a case in which the massage apparatus 100 is providing massage to the user, one of the function buttons 1822 and 1823 may be a button for stopping the provision of massage. However, in a case in which the massage apparatus 100 is not providing massage, one of the function buttons 1822 and 1823 may be changed into a button for executing provision of massage. As described above, since the function defined to the function button may be changed when the operation state of the massage apparatus is changed, the massage apparatus 100 according to the present invention can control various functions suitable for the current situation by using a small number of buttons.

According to an embodiment of the present invention, in a case in which the user presses one of the function buttons 1822 and 1823, the massage apparatus 100 may change an angle of the chair. More specifically, in a case in which the user presses the first function button 1822, the massage apparatus 100 may change an angle of the chair. The massage apparatus 100 may continuously change the angle while the user presses the first function button 1822. Moreover, the massage apparatus 100 may move at a predetermined angle whenever the user presses the first function button 1822. The massage apparatus 100 may repeat the operation that gradually lays down the chair and gradually makes the chair stand when the user presses the first function button 1822. The user can stop the angle control by pulling the hand away from the first function button 1822 when the chair reaches a desired angle.

According to an embodiment of the present invention, in a case in which the user presses the other function button in a state in which the massage apparatus 100 stops, the massage apparatus 100 may start to provide massage to the user based on a recommended massage mode. The recommended massage mode may be previously stored in the massage apparatus 100. Here, the other function button may be the second function button 1823. In addition, in a case in which the user presses the other button in a state in which the massage apparatus 100 is moving, the massage apparatus 100 may temporarily stop the massage mode. The massage apparatus 100 may temporarily stop all operations thereof, but is not limited thereto. The massage apparatus 100 may stop only some operations. For instance, the massage apparatus 100 may temporarily stop only the expansion/contraction of the arm airbag, but may continuously perform other massage operations. In a case in which the user presses the other button in a state in which the massage apparatus 100 is temporarily stopped, the massage apparatus 100 may restart the massage operation. For instance, if the entire operations of the massage apparatus are temporarily stopped, the massage apparatus 100 may restart the entire operations. Alternatively, if only some operations of the massage apparatus 100 are temporarily stopped, the massage apparatus 100 may restart the some operations. For example, if only the arm airbag operation is temporarily stopped, the massage apparatus 100 may restart the arm airbag operation.

In addition, according to an embodiment of the present invention, in a case in which the user presses any one among the function buttons 1822 and 1823, the massage mode may be ended. That is, the massage apparatus 100 may not provide massage to the user. Additionally, the massage apparatus 100 may return to the basic state.

According to another embodiment of the present invention, in a case in which the user presses one of the function buttons 1822 and 1823, the massage apparatus 100 may change an angle of the chair. More specifically, in a case in which the user presses the first function button 1822, the massage apparatus 100 may change an angle of the chair. The massage apparatus 100 may continuously change the angle while the user presses the first function button 1822. Moreover, the massage apparatus 100 may move at a predetermined angle whenever the user presses the first function button 1822. The massage apparatus 100 may repeat the operation that gradually lays down the chair and gradually makes the chair stand when the user presses the first function button 1822. The user can stop the angle control by pulling the hand away from the first function button 1822 when the chair reaches a desired angle.

According to another embodiment of the present invention, in a case in which the user presses the other function button in a state in which the massage apparatus 100 stops, the massage apparatus 100 may start to provide massage to the user based on a recommended massage mode. The recommended massage mode may be previously stored in the massage apparatus 100. Here, the other function button may be the second function button 1823. In addition, in a case in which the user presses the other button in a state in which the massage apparatus 100 is moving, the massage apparatus 100 may temporarily stop the massage mode. The massage apparatus 100 may temporarily stop all operations thereof, but is not limited thereto. The massage apparatus 100 may stop only some operations. For instance, the massage apparatus 100 may temporarily stop only the expansion/contraction of the arm airbag, but may continuously perform other massage operations. In a case in which the user presses the other button in a state in which the massage apparatus 100 is temporarily stopped, the massage apparatus 100 may restart the massage operation. For instance, if the entire operations of the massage apparatus are temporarily stopped, the massage apparatus 100 may restart the entire operations. Alternatively, if only some operations of the massage apparatus 100 are temporarily stopped, the massage apparatus 100 may restart the some operations. For example, if only the arm airbag operation is temporarily stopped, the massage apparatus 100 may restart the arm airbag operation.

In addition, according to another embodiment of the present invention, in a case in which the user presses both of the function buttons 1822 and 1823 long, the massage mode may be ended. That is, the massage apparatus 100 may not provide a massage to the user. Furthermore, the massage apparatus 100 may return to the basic state. Till now, the predetermined operations of the function buttons 1822 and 1823 have been described. However, the present invention is not limited thereto. The massage apparatus 100 can set functions of the function buttons 1822 and 1823 based on the use's selection. For instance, the massage apparatus 100 may display a menu for setting the function buttons 1822 and 1823 on a display. The massage apparatus 100 may receive the user's input to show which operation will be executed according to patterns that the user presses the function buttons 1822 and 1823. The pressed pattern of the function buttons 1822 and 1823 may include "a case in which the first function button 1822 and the second function button 1823 are simultaneously pressed shortly once", "a case in which the first function button 1822 and the second function button 1823 are simultaneously pressed shortly twice", or "a case in which the first function button 1822 and the second function button 1823 are simultaneously pressed long". Moreover, pressed pattern of the function buttons 1822 and 1823 may include a case in which the first function button 1822 is pressed shortly and the second function button 1823 is pressed shortly in a fixed time. Based on the received input by the user, the massage apparatus 100 may determine which operation will be executed when the function buttons 1822 and 1823 are pressed in the specific pattern by the user.

Referring to FIGS. 18 and 19, the first bio-signal measurement unit 510 may include light guides 1811, 1812, 1813 and 1814. The light guides 1811, 1812, 1813 and 1814 may have a configuration to transmit light emitted from the plurality of LEDs 1941 and 1942 to the user's hand.

The light guides 1811, 1812, 1813 and 1814 may be made of a transparent material. Moreover, the light guides 1811, 1812, 1813 and 1814 may be made of a material that selectively transmits the wavelength of the light emitted from the plurality of LEDs 1941 and 1942.

The light guide 1811 may be arranged on the wrist. Due to the light guide 1811, the user can prevent tunnel syndrome by applying the light emitted from the LEDs 1941 to the wrist. Furthermore, the light guides 1811, 1812, 1813 and 1814 may be arranged on a portion of the user's fingers. Due to the light guides 1811, 1812, 1813 and 1814, the user may prevent diseases which may be caused to finger joints by applying the light emitted from the plurality of LEDs 1942 for treatment.

Referring to FIG. 19, the upper cover 970 may include a palm support 1971 and an upper cover fixing portion 1972. The palm support 1971 may have holes for inserting the light guides 1812, 1813 and 1814 thereinto. Moreover, the upper cover fixing portion 1972 may have a hole for inserting the light guide 1811 thereinto.

The first bio-signal measurement unit 510 may further include a heating unit support 1950. The heating unit support may be fixed on the lower cover 920. The heating unit support 1950 may be located below a heating unit to maintain the shape of the heating unit. The heating unit support 1950 may have an upwardly convex shape. In a case in which the user naturally puts the hand on the first bio-signal measurement unit 510, when viewed from the user's palm, the user's palm may be concave. Accordingly, the heating unit support 1950 may be upwardly (1902) convex to effectively transmit heat to the user's palm. In addition, as illustrated in FIG. 19, the upper surface of the heating unit support 1950 may be flat.

The heating unit support 1950 may be arranged above (1902) the plurality of LEDs 1941 and 1942 for treatment. The heating unit support 1950 may be made of a transparent material not to hide light of the LEDs 1941 and 1942.

The first bio-signal measurement unit 510 may further include a heating unit 1960. The heating unit 1960 may be fixed on the heating unit support 1950. The heating unit 1960 may be put on the flat portion of the upper surface of the heating unit support 1950. The heating unit 1960 may generate heat based on a control signal of the control unit 300. The heating unit 1960 may provide thermal fomentation to the user.

The heating unit 1960 may be located above (1902) the plurality of LEDs. The heating unit 1960 may have a shape not to hide the light of the LEDs 1941 and 1942. For instance, the heating unit 1960 may include at least one slit. Due to the slit, light of the LEDs 1941 and 1942 is not blocked by the heating unit 1960, and can be transmitted to the user's palm. In addition, the at least one slit included in the heating unit 1960 may be arranged at lower ends of the light guides 1811, 1812, 1813 and 1814. Referring to FIG. 19, the heating unit 1960 may have three slits formed in the back-and-forth direction for the light guides 1811, 1812, 1813 and 1814. The light of the LEDs 1942 may be transmitted to the user's palm due to the three slits formed in the back-and-forth direction. Moreover, the heating unit 1960 may have one slit formed in the horizontal direction for the light guide 1811. Due to the slit formed in the horizontal direction, the light of the plurality of LEDs 1941 may be transmitted to the user's wrist.

The first bio-signal measurement unit 510 may include a plurality of LEDs 1941 and 1942 for treatment. The plurality of LEDs 1941 and 1942 may be fixed on the lower cover 920. The plurality of LEDs 1941 and 1942 for treatment may emit light based on a control signal of the control unit 300. The plurality of LEDs 1941 and 1942 for treatment may have a configuration to apply LED light to the user's wrist. Additionally, the plurality of LEDs 1941 and 1942 for treatment may have a configuration to apply LED light to the user's fingers.

The plurality of LEDs 1941 and 1942 for treatment may be located on an LED control PCB 1940. The LED control PCB 1940 may include a processor and a memory. The control unit 300 may send a control signal to the LED control PCB 1940. The LED control PCB 1940 may turn on or off at least a portion of the LEDs 1941 and 1942 based on the control signal.

The first bio-signal measurement unit 510 may include a lower cover 920. The lower cover 920 may be placed on the housing of the arm massage unit. The lower cover 920 may be fixed on the housing of the arm massage unit, but is not limited thereto. For example, the lower cover 920 may not be fixed on the housing of the arm massage unit. The lower cover 920 may be slidable on the housing of the arm massage unit.

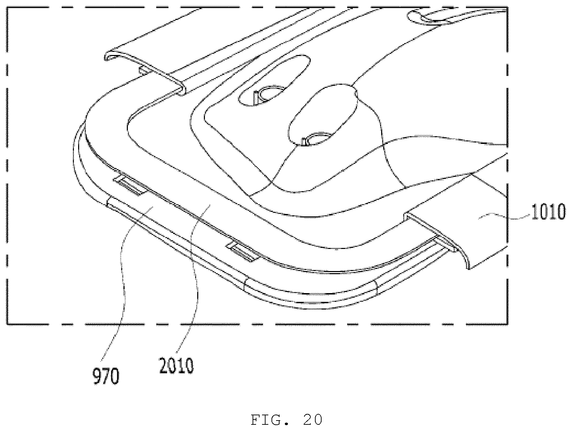

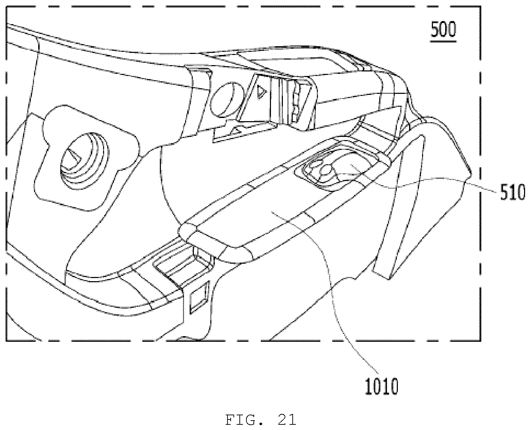

FIG. 20 is a view for depicting a first bio-signal measurement unit according to an embodiment of the present invention. FIG. 21 is a view for depicting a first bio-signal measurement unit according to an embodiment of the present invention.

Although FIG. 20 illustrates an embodiment different from that of FIG. 9, some of components of FIG. 20 may be duplicated with those of FIG. 9. FIG. 20 may be the same as FIGS. 18 and 19. Components which are not described in conjunction with FIG. 20 may be described in conjunction with FIG. 9.

FIG. 20 illustrates that a portion of a cover sheet 1010 is controlled for the convenience of description. Additionally, FIG. 20 does not illustrate an electrode inserted into the upper cover 970.

In FIG. 20, a plate 2010 may be disposed on the upper cover 970. The plate may be fixed on the cover sheet 1010 of the arm massage unit 500. That is, at least a portion of the plate 2010 may be surrounded by the cover sheet 1010. Moreover, the whole of the plate 2010 may be surrounded by the cover sheet 1010. FIG. 20 illustrates that a portion of the plate 2010 is not surrounded by the cover sheet 1010, but it is because a portion of the cover sheet 1010 is not illustrated for the convenience of description.

The plate 2010 may be located on the upper cover 970. More specifically, the plate 2010 may be located on an upper cover fixing portion 1972 of the upper cover 970. The plate 2010 may be arranged along the upper cover fixing portion 1972. A portion of the cover sheet 1010 may be located between the upper cover 970 and the plate 2010. The plate 2010 may be made of a metal material. At least two magnets 930 may face the plate 2010.

Referring to FIGS. 19 and 20, the magnets 930 located below the upper cover 970 may generate a magnetic force with the plate 2010. The plate 2010 and the upper cover 970 may come into contact with each other by the magnetic force between the magnets 930 and the plate 2010. In addition, due to the magnetic force between the magnets 930 and the plate 2010, it is prevented that the cover sheet 1010 surrounding the plate 2010 is separated from the plate 2010. Therefore, according to the present invention, it is prevented that the cover sheet 1010 becomes crumpled.

Due to the magnetic force between the magnets 930 and the plate 2010, the first bio-signal measurement unit 510 may not move horizontally. Furthermore, due to the cover sheet 1010, the first bio-signal measurement unit 510 may not move horizontally and vertically. FIG. 21 illustrates an example in which the first bio-signal measurement unit 510 is located below the cover sheet 1010 according to FIG. 20.

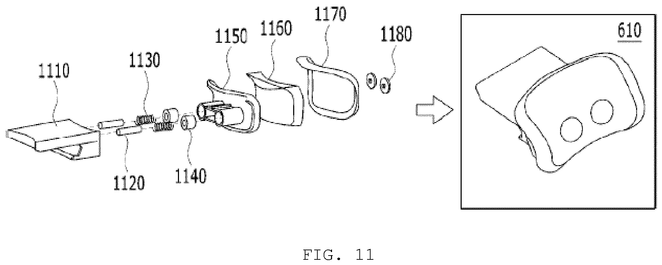

FIG. 11 illustrates a second bio-signal measurement unit according to an embodiment of the present invention.

The second bio-signal measurement unit 610 may include a leg support 1110. Referring to FIGS. 6 and 11, the leg support 1110 may be formed on the housing of the foot massage unit 622 to be located at the back of the leg of the user. Here, the back may mean the back side when the user sits on the massage apparatus 100.

The second bio-signal measurement unit 610 may include a foot holder 1150. The foot holder 1150 may be coupled to the front of the leg support 1110. The foot holder 1150 may support the user's leg. Furthermore, the foot holder 1150 may have a concave shape toward the user so that the user can feel comfortable when putting the leg on the foot holder 1150.

The second bio-signal measurement unit 610 may include an elastic layer 1160. The elastic layer 1160 may be attached to the front of the foot holder 1150. The elastic layer 1160 may be made of an elastic material. Due to the elastic layer 1160, the user may feel comfortable even if the user's leg touches the electrode.

The second bio-signal measurement unit 610 may include a cover layer 1170. The cover layer 1170 may be attached on the front of the elastic layer 1160. The cover layer 1170 may be a component for providing the user with a clean design. The cover layer 1170 may be made of leather, artificial leather or polyurethane.

The second bio-signal measurement unit 610 may include a second bio-signal measurement electrode 118. The second bio-signal measurement electrode 1180 may be inserted into a hole formed in the front of the cover layer. In addition, the second bio-signal measurement electrode 1180 may be a silicon electrode.

The control unit 300 may measure the user's bio-signal by using at least one among the first bio-signal measurement electrode 980 and the second bio-signal measurement electrode 610. The second bio-signal measurement electrode 1180 may get in contact with the user. The second bio-signal measurement electrode may be electrically connected to the control unit 300. The control unit 300 may acquire a bio-signal based on a signal from the second bio-signal measurement electrode 1180.

An electric wire may be connected from the second bio-signal measurement electrode 1180 to the control unit 300 so that the second bio-signal measurement electrode 1180 is electrically connected with the control unit 300. The leg support 1110, the foot holder 1150, the elastic layer 1160, and a cover layer 1170 may respectively have holes for wiring. The user may put the leg on the bio-signal measurement unit 610. In this instance, the foot holder 1150, the elastic layer 1160, and the cover layer 1170 may move forward and backward with respect to the leg support 1110. Due to the movement, an electric wire holder of an elastic material (not illustrated) may hold the electric wire so that the electric wire between the second bio-signal measurement electrode 1180 and the control unit 300 is not damaged. The electric wire holder may be fixed at any one among the foot holder 1150, the elastic layer 1160, and the cover layer 1170. The electric wire holder may enhance durability of the massage apparatus 100.

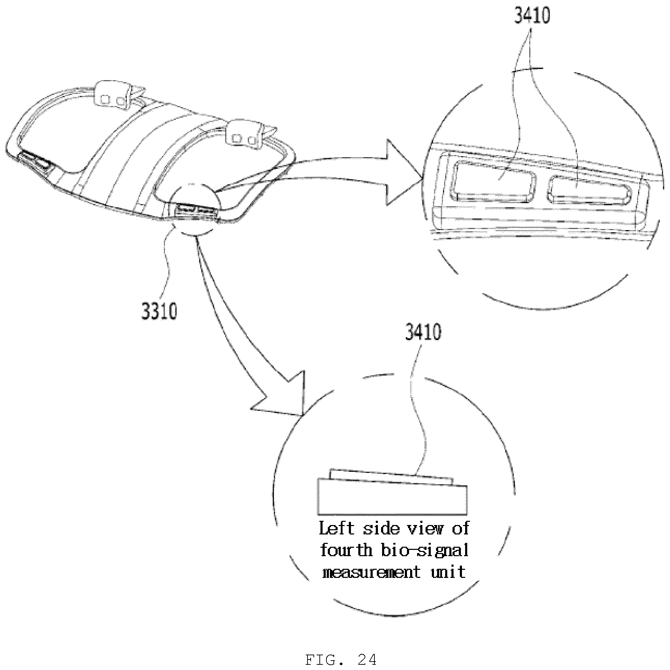

Referring to FIG. 24, the fourth bio-signal measurement unit 3310 may include a fourth bio-signal measurement electrode 3410. FIG. 24 is a left side view of the bio-signal measurement unit 3310. The upper surface of the fourth bio-signal measurement electrode 3410 faces the user, and is at an angle of 1 to 45 degrees to the ground surface. Specifically, in a case in which the leg massage unit 2300 of the massage apparatus 100 is in the basic state, the upper surface of the fourth bio-signal measurement electrode 3410 faces the user, and is at an angle of 1 to 45 degrees to the ground surface. The basic state is a posture of the massage apparatus 100 when the massage apparatus 100 does not provide massage, and may mean a state in which the user can easily sit on the massage apparatus 100. Furthermore, the basic state may be a state in which the leg massage unit 2300 is nearly perpendicular to the ground surface.

In addition, the fourth bio-signal measurement electrode 3410 may be a silicon electrode. The control unit 300 may measure the user's bio-signal by using at least one among the third bio-signal measurement electrode 3220, the second bio-signal measurement electrode 1180, and the fourth bio-signal measurement electrode 3410. The fourth bio-signal measurement electrode 3410 may get in contact with the user. The fourth bio-signal measurement electrode 3410 may be electrically connected with the control unit 300. The control unit 300 may acquire the bio-signal based on the signal from the fourth bio-signal measurement electrode 3410. In order to electrically connect the fourth bio-signal measurement electrode 3410 to the control unit 300, an electric wire may be connected from the fourth bio-signal measurement electrode 3410 to the control unit 300.

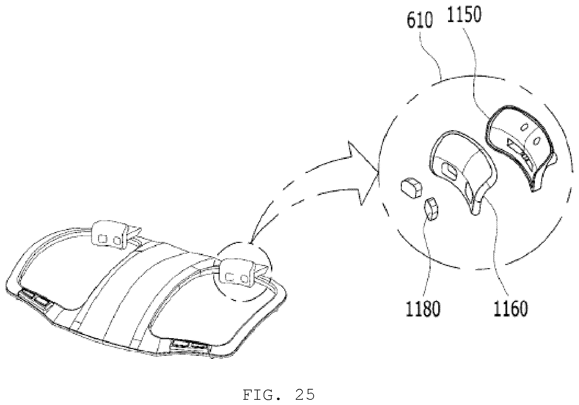

FIG. 25 illustrates a second bio-signal measurement unit according to an embodiment of the present invention.

FIG. 25 illustrates a portion of the components of the second bio-signal measurement unit 610. Components which are not illustrated in FIG. 25 may be the same as FIG. 11.

According to various embodiments of the present invention, the second bio-signal measurement unit 610 may include a foot holder 1150. The foot holder 1150 may be coupled to the front of the leg support 1110. The foot holder 1150 may support the user's leg. In addition, the foot holder 1150 may have a concave shape toward the user so that the user feels comfortable when the user puts the foot on the foot holder 1150. Additionally, the foot holder 1150 may be injection-molded with silicon. Since the foot holder 1150 is made of a soft material, the user may feel comfortable even when the second bio-signal measurement unit 610 touches the user's foot.

The second bio-signal measurement unit 610 may include an elastic layer 1160. The elastic layer 1160 may be attached to the front of the foot holder 1150. The elastic layer 1160 and the foot holder 1150 may be bonded to each other using double sided tape. The elastic layer 1160 may be made of an elastic material. For instance, the elastic layer 1160 may be made of a silicon material. Moreover, the elastic layer 1160 may have a hardness of HS40 or less. Even if the user's leg touches the electrode, the user may feel softness due to the elastic layer 1160.

The second bio-signal measurement unit 610 may not include the cover layer 1170 in order to prevent the cover layer 1170 from interfering between the user's foot and the electrode.

The second bio-signal measurement unit 610 may include a second bio-signal measurement electrode 1180. The second bio-signal measurement electrode 1180 may be inserted into a hole formed in the elastic layer 1160. In addition, the second bio-signal measurement electrode 1180 may be a silicon electrode.

The second bio-signal measurement electrode 1180 may have a thickness of 5.0 to 7.0 mm. In addition, the horizontal and vertical lengths of the second bio-signal measurement electrode 1180 may range from 13 mm to 17 mm. The second bio-signal measurement electrode 1180 having such a size can most accurately measure a bio-signal.

The second bio-signal measurement electrode 1180 may be connected to a cable by an internal snap button. The control unit 300 may measure the user's bio-signal using the second bio-signal measurement electrode 1180.

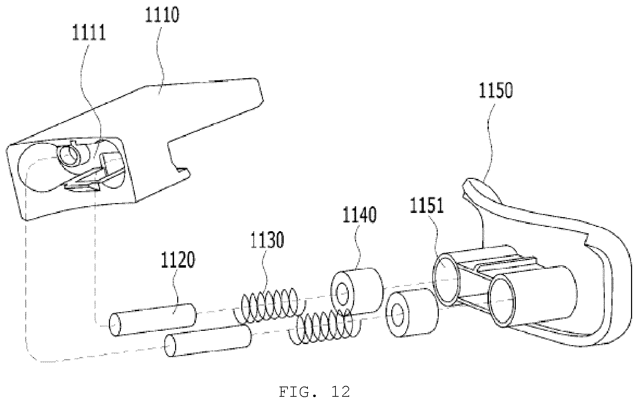

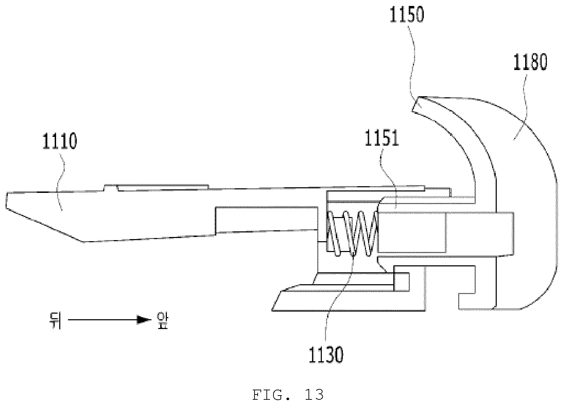

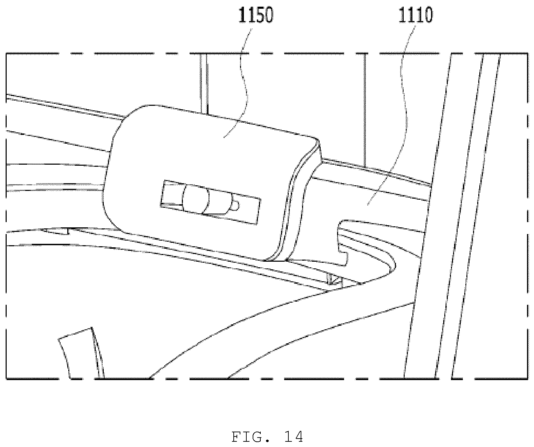

FIG. 12 is a view for depicting a second bio-signal measurement unit according to an embodiment of the present invention. FIG. 13 is a view for depicting a second bio-signal measurement unit according to an embodiment of the present invention. FIG. 14 is a view for depicting a second bio-signal measurement unit according to an embodiment of the present invention.

Referring to FIGS. 12 to 14, the foot holder 1150 may include a coupling protrusion 1151. The coupling protrusion 1151 may be inserted into a coupling groove 1111 of the leg support 1110. The coupling protrusion 1151 may have a groove. A guide ring 1140 may be inserted into the groove of the coupling protrusion 1151.

A guide pin 1120 may be inserted into a spring 1130. Moreover, the guide pin 1120 may be inserted into the groove of the guide ring 1140. The spring 1130 may not be inserted into the groove of the guide ring 1140. The spring 1130 may receive a force by one side of the guide ring 1140.

The guide pin 1120, the spring 1130, the guide ring 1140, and the coupling protrusion 1151 of the foot holder 1150 may be inserted into the coupling groove 1111 of the leg support 1110. The spring 1130 may receive a force by one side of the coupling groove 111 and one side of the guide ring 1140 to be compressed. The spring 1130 may push the guide ring 1140 forward. Therefore, the second bio-signal measurement electrode 1180 may easily touch the back of the leg of the user.

The massage apparatus 100 may include two first bio-signal measurement units 510. For instance, the massage apparatus 100 may include a first bio-signal measurement unit of the left side and a first bio-signal measurement unit of the right side. Each of the first bio-signal measurement unit of the left side and the first bio-signal measurement unit of the right side may include two first bio-signal measurement electrodes.

The massage apparatus 100 may include two second bio-signal measurement units 610. For instance, the massage apparatus 100 may include a second bio-signal measurement unit of the left side and a second bio-signal measurement unit of the right side. Each of the second bio-signal measurement unit of the left side and the second bio-signal measurement unit of the right side may include two second bio-signal measurement electrodes.

The massage apparatus 100 may include two third bio-signal measurement units 3210. For instance, the massage apparatus 100 may include a third bio-signal measurement unit of the left side and a third bio-signal measurement unit of the right side. Each of the third bio-signal measurement unit of the left side and the third bio-signal measurement unit of the right side may include two third bio-signal measurement electrodes.

The massage apparatus 100 may include two fourth bio-signal measurement units 3310. For instance, the massage apparatus 100 may include a fourth bio-signal measurement unit of the left side and a fourth bio-signal measurement unit of the right side. Each of the fourth bio-signal measurement unit of the left side and the fourth bio-signal measurement unit of the right side may include two fourth bio-signal measurement electrodes.

The control unit 300 may measure the user's bio-signal by using at least two among the four first bio-signal measurement electrodes, at least two among the four second bio-signal measurement electrodes, at least two among the four third bio-signal measurement electrodes, and at least two among the four fourth bio-signal measurement electrodes. Therefore, the massage apparatus 100 may accurately measure a bio-signal. In addition, the control unit 300 may acquire information on the user's physical condition based on the measured bio-signal. A general bio-signal measurer may ask a user to take a special posture because the measurer cannot properly measure a bio-signal if the user's arms touch the body or the legs touch each other. However, in a case in which the user sits or lies on the massage apparatus 100 according to the present invention, the user can take the optimum posture to measure a bio-signal due to the housing of the massage apparatus 100. Therefore, the massage apparatus 100 according to the present invention can measure a bio-signal the most accurately.

In addition, the massage apparatus 100 may periodically measure a bio-signal while providing massage. Accordingly, the user's health improvement effect according to the massage can be displayed. Moreover, the massage apparatus 100 may cumulatively store the measured bio-signals of the user or information on the physical condition, or may transfer the measured bio-signals or information on the physical condition to a cloud server. The measured bio-signals of the user or information on the physical condition may be used for diagnosis of the user later.

Furthermore, the massage apparatus 100 can acquire information on a plurality of bio-signals or a plurality of physical conditions by periodically performing measurement while providing massage once. Providing massage once may mean that the massage apparatus 100 provides one massage mode to the user. The massage apparatus 100 may acquire information on a representative bio-signal or a representative physical condition out of information on the plurality of bio-signals or the plurality of physical conditions. For instance, the massage apparatus 100 may acquire at least one among an average value, a median value, a variance, or a standard deviation of information on the plurality of bio-signals or the plurality of physical conditions, and may use the acquired value as information on the representative bio-signal or the representative physical condition. Accordingly, the user can easily understand the characteristics of the bio-signal or the characteristics of the information on the physical condition by number of times of massages.

In addition, the massage apparatus 100 may represent bio-signals over time or information on physical conditions on a display in a graph on the basis of information on a plurality of bio-signals or a plurality of physical conditions. The massage apparatus 100 may represent information on a representative bio-signal or representative physical condition according to the number of massages on a display in a graph on the basis of a plurality of bio-signals or information on a plurality of physical conditions.

FIG. 24 is a view illustrating a fourth bio-signal measurement unit 3310 according to an embodiment of the present invention.

The control unit 300 may measure the user's bio-signal by using at least one among the first bio-signal measurement electrode 980, the second bio-signal measurement electrode 1180, the third bio-signal measurement electrode 3220, and the fourth bio-signal measurement electrode 3410. The fourth bio-signal measurement electrode 3410 may come into contact with the user. The fourth bio-signal measurement electrode 3410 may be electrically connected to the control unit 300. In order to electrically connect the fourth bio-signal measurement electrode 3410 to the control unit 300, an electric wire may be connected from the fourth bio-signal measurement electrode 3410 to the control unit 300. Based on the signal from the fourth bio-signal measurement electrode 3410, the control unit 300 may obtain a bio-signal.

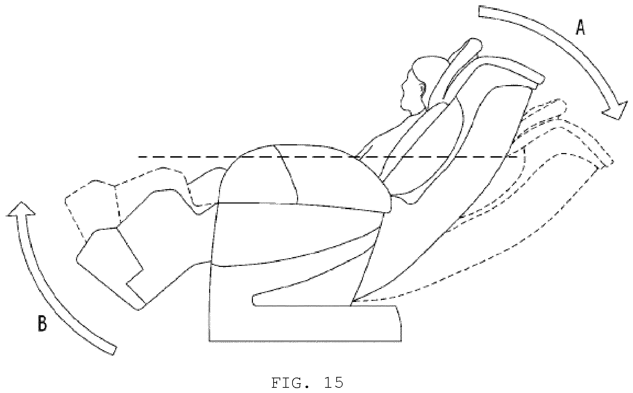

FIG. 15 is a view for depicting an example in which a posture of the massage apparatus is adjusted to measure a bio-signal according to an embodiment of the present invention.

Referring to FIG. 15, the control unit 300 may control the operation of the first bio-signal measurement unit 510, the second bio-signal measurement unit 610, the third bio-signal measurement unit 3210, or the fourth bio-signal measurement unit 3310. The control unit 300 may perform bio-signal measurement after adjusting the posture of the massage apparatus 100 in a case in which the user inputs measurement of information on the physical condition.

Moreover, the massage apparatus 100 may automatically measure the user's bio-signal. While the massage apparatus 100 provides massage to the user according to the massage mode, the control unit 300 may measure the user's bio-signal at a predetermined cycle by using at least two among the first bio-signal measurement unit 510, the second bio-signal measurement unit 610, the third bio-signal measurement unit 3210, and the fourth bio-signal measurement unit 3310. Furthermore, the control unit 300 may periodically obtain information on the user's physical condition based on the measured bio-signal. The massage apparatus 100 may adjust the posture of the massage apparatus 100 according to the massage mode. In a case in which the posture of the massage apparatus 100 is suitable for measuring bio-signals, the control unit 300 may measure the user's bio-signal by using at least two among the first bio-signal measurement unit 510, the second bio-signal measurement unit 610, the third bio-signal measurement unit 3210, and the fourth bio-signal measurement unit 3310. If the user is sitting or lying on the massage apparatus 100, it may always be a suitable posture for measuring a bio-signal, so the control unit 300 may periodically measure bio-signals while the massage apparatus 100 provides massage. In addition, the control unit 300 may periodically obtain information on the user's physical condition based on the bio-signal. The information on the physical condition may include at least one among body composition information and electrocardiogram information.

The control unit 300 may simultaneously measure body composition information and electrocardiogram information of the user by using at least two among the first bio-signal measurement unit 510, the second bio-signal measurement unit 610, the third bio-signal measurement unit 3210, and the fourth bio-signal measurement unit 3310. Signals for obtaining body composition information and electrocardiogram information may be similar signals. The control unit 300 may simultaneously measure body composition information and electrocardiogram information of the user by analyzing signals received from at least two among the first bio-signal measurement unit 510, the second bio-signal measurement unit 610, the third bio-signal measurement unit 3210, and the fourth bio-signal measurement unit 3310. It may take about 15 seconds for the massage apparatus 100 to measure body composition by using at least one among the first bio-signal measurement unit 510, the second bio-signal measurement unit 610, the third bio-signal measurement unit 3210, and the fourth bio-signal measurement unit 3310. In addition, it may take about one minute for the massage apparatus 100 to measure electrocardiogram by using at least one among the first bio-signal measurement unit 510, the second bio-signal measurement unit 610, the third bio-signal measurement unit 3210, and the fourth bio-signal measurement unit 3310. The control unit 300 may simultaneously measure body composition information and electrocardiogram information by using at least a portion of the plurality of electrodes included in the first bio-signal measurement unit 510, the second bio-signal measurement unit 610, the third bio-signal measurement unit 3210, or the fourth bio-signal measurement unit 3310.

However, the present invention is not limited thereto. The control unit 300 may measure at least one among body composition information and electrocardiogram information at a predetermined cycle by using at least one among the first bio-signal measurement unit 510, the second bio-signal measurement unit 610, the third bio-signal measurement unit 3210, and the fourth bio-signal measurement unit 3310. The cycle for obtaining body composition information and the cycle for obtaining electrocardiogram information may be different from each other or may be the same.

The control unit 300 may cumulatively store the measured bio-signals of the user or information on the physical condition of the user. Moreover, the control unit 300 may transmit the bio-signals of the user and/or information on the physical condition of the user to a cloud server and/or a hospital server with the user's approval. Accordingly, the user can check the process of changing the physical condition of the user. In addition, when a medical person later diagnoses the user's health condition, the cumulatively stored bio-signals or information on the physical condition may be used. Therefore, the user's health condition can be accurately diagnosed.

First embodiment of posture adjustment of massage apparatus 100

According to an embodiment of the present invention, the massage apparatus 100 may measure a bio-signal in a state in which the leg massage unit 2300 of the massage apparatus 100 is not lifted. The state in which the leg massage unit 2300 is not lifted means a state in which the angle between the leg massage unit 2300 and the vertical line of the ground is less than a predetermined angle. Furthermore, the massage apparatus 100 may measure a bio-signal in a state in which the body massage unit 2100 of the massage apparatus 100 is not tilted. The state in which the body massage unit 2100 is not tilted may mean a state in which the user is sitting on the massage apparatus 100 with the waist erected. Even if the leg massage unit 2300 is not lifted, when the user puts the sole of the foot on the fourth bio-signal measurement unit 3310 located on the upper surface of the housing of the leg massage unit, the fourth bio-signal measurement unit 3310 comes into close contact with the user's sole so that the bio-signal may be measured more accurately. In addition, according to an embodiment of the present invention, the massage apparatus 100 may measure a bio-signal without tilting the body massage unit 2100 backward. In a case in which the body massage unit 2100 is not tilted backward and the leg massage unit 2300 is not lifted, the massage apparatus 100 may measure a bio-signal from the user's palm by using one among the first bio-signal measurement unit 510 and the second bio-signal measurement unit 3210, and may measure a bio-signal from the user's sole of the foot by using the fourth bio-signal measurement unit.

Second embodiment of posture adjustment of massage apparatus 100

According to an embodiment of the present invention, the massage apparatus 100 may measure a bio-signal after the leg massage unit 2300 of the massage apparatus 100 is lifted (B). In the second embodiment, the angle between the leg massage unit 2300 and the vertical line of the ground may be more than a predetermined angle. For instance, the massage apparatus 100 may operate the leg angle actuator provided in the body massage unit 2100 to lift the leg massage unit 2300, and the control unit 300 measure a bio-signal after the leg massage unit 2300 is lifted. In a case in which the massage apparatus 100 lifts the leg massage unit 2300 and the angle between the leg massage unit 2300 and the vertical line of the ground is more than a predetermined angle, the second bio-signal measurement unit 610 located at the back of the user's leg comes into close contact with the user's leg so that the bio-signal can be more accurately measured. That is, in a case in which the massage apparatus 100 does not tilt the body massage unit 2100 backward and lifts the leg massage unit 2300, the massage apparatus 100 may measure a bio-signal from the user's hand by using one among the first bio-signal measurement unit 510 and the second bio-signal measurement unit 3210, and may measure a bio-signal from the user's heel by using the second bio-signal measurement unit 610.

Third embodiment of posture adjustment of massage apparatus 100

According to an embodiment of the present invention, the massage apparatus 100 may measure a bio-signal after tilting the body massage unit 2100 backward (A). For instance, the massage apparatus 100 may operate the back angle actuator included in the body massage unit 2100 to tilt the body massage unit 2100 backward, and the control unit 300 may measure a bio-signal after tilting the body massage unit 2100 backward. In a case in which the body massage unit 2100 is tilted backward, the user's hand may move backward. As described above, the first bio-signal measurement unit 510 may move forward and backward. Accordingly, in a case in which the body massage unit 2100 is tilted backward, the first bio-signal measurement unit 510 moves backward, so that the first bio-signal measurement unit 510 is located below the user's palm. Therefore, the massage apparatus 100 may measure the user's bio-signal accurately. In addition, the third bio-signal measurement unit 3210 is fixed on the arm massage unit, but may come into contact with the user's palm even if the body massage unit 2100 is tilted backward since the third bio-signal measurement unit 3210 has a horizontally long electrode. Therefore, the massage apparatus 100 may accurately measure the user's bio-signal. Additionally, in the third embodiment, the leg massage unit 2300 may not be lifted. As described above, in this case, when the user puts the sole on the fourth bio-signal measurement unit 3310 located on the upper surface of the housing of the leg massage unit, the fourth bio-signal measurement unit 3310 comes into contact with the user's sole so that the bio-signal may be accurately measured. To sum up, in the case in which the body massage unit 2100 is tilted backward and the leg massage unit 2300 is not lifted, the massage apparatus 100 may measure a bio-signal from the user's palm by using one among the first bio-signal measurement unit 510 and the second bio-signal measurement unit 3210, and may measure a bio-signal from the user's sole by using the fourth bio-signal measurement unit 3310.

Fourth embodiment of posture adjustment of massage apparatus 100

According to an embodiment of the present invention, the massage apparatus 100 may lift the leg massage unit 2300 of the massage apparatus 100 (B), and measure a bio-signal after tilting the body massage unit 2100 backward (A). For instance, the massage apparatus 100 may operate the leg angle actuator included in the body massage unit 2100 to lift the leg massage unit 2300, operate the back angle actuator to tilt the body massage unit 2100 backward, and then, measure a bio-signal. In the case in which the body massage unit 2100 is tilted backward and the leg massage unit 230 is lifted, the massage apparatus 100 may measure a bio-signal from the user's palm by using one among the first bio-signal measurement unit 510 and the second bio-signal measurement unit 3210, and may measure a bio-signal from the user's heel by using the fourth bio-signal measurement unit 3310.

According to an embodiment of the present invention, the massage apparatus 100 may determine whether to measure a bio-signal based on the massage mode. The user may do not want that the user's biometric information is acquired unnecessarily. The user may select a massage mode not to acquire the bio-signal so that the massage apparatus 100 does not measure the bio-signal.

The control unit 300 may periodically measure bio-signals from the first bio-signal measurement unit 510, the second bio-signal measurement unit 610, the third bio-signal measurement unit 3210, or the fourth bio-signal measurement unit 3310. The control unit 300 may determine an electrode which does not get in contact with the user's skin based on the signal received from the first bio-signal measurement unit 510, the second bio-signal measurement unit 610, the third bio-signal measurement unit 3210, or the fourth bio-signal measurement unit 3310. In a case in which there is an electrode determined as the electrode which does not get in contact with the user's skin, the control unit 300 may generate an alarm signal. The alarm signal may include information on the location of the electrode which does not get in contact with the user's skin. The control unit 300 can control the alarm signal to be output through the output unit. The alarm signal may be output into light or sound. The user can adjust the posture based on the alarm signal so that the skin comes into contact with the electrode.

In a case in which it is determined that a portion of the electrode included in the first bio-signal measurement unit 510, the second bio-signal measurement unit 610, the third bio-signal measurement unit 3210, or the fourth bio-signal measurement unit 3310 does not get in contact with the user's skin, the control unit 300 may control to measure a bio-signal just by the electrode getting in contact with the skin. More specifically, in a case in which it is determined that a portion of the electrode included in the first bio-signal measurement unit 510, the second bio-signal measurement unit 610, the third bio-signal measurement unit 3210, or the fourth bio-signal measurement unit 3310 does not get in contact with the user's skin, the control unit 300 may output an alarm signal, and wait for a predetermined period of time till a portion of the electrode included in the first bio-signal measurement unit 510, the second bio-signal measurement unit 610, the third bio-signal measurement unit 3210, or the fourth bio-signal measurement unit 3310 gets in contact with the user's skin. Even after the predetermined period of time, if it is determined that a portion of the electrode included in the first bio-signal measurement unit 510, the second bio-signal measurement unit 610, the third bio-signal measurement unit 3210, or the fourth bio-signal measurement unit 3310 does not get in contact with the user's skin, the control unit 300 may control to measure a bio-signal just by the electrode getting in contact with the skin. Even though the user does not see or hear the alarm signal, the massage apparatus 100 may accurately measure the bio-signal and obtain information on the user's physical condition. The control unit 300 may store the information on the user's physical condition in the memory. In addition, the control unit 300 may store also information on the position of the used electrode in the memory in order to obtain information on the user's physical condition.

Various embodiments according to the present invention have been described. It will be understood by those skilled in the art that the present invention may be implemented in a modified form without departing from the essential characteristics of the present invention. Therefore, the disclosed embodiments should be considered in the expository perspective rather than limitative perspective. Therefore, the scope of the present invention is defined not by the detailed description of the invention but by the appended claims, and all differences within the equivalent scope will be construed as being included in the present invention.

Meanwhile, embodiments of the present invention described above may be made as a computer-executable program, and may be implemented in a general-purpose digital computer that operates the program using a computer-readable recording medium. The computer-readable recording medium includes a storage medium, such as a magnetic storage medium (e.g., a ROM, a floppy disk, a hard disk, etc.), or an optical recording medium (e.g., a CD-ROM, a DVD, etc.).

## Claims

1. A massage apparatus comprising:
a first bio-signal measurement unit which is formed in an arm massage unit, is located at a portion on which a user's palm is put, is movable forward and backward on the basis of the position of the hand of the user, and includes at least one electrode;
a second bio-signal measurement unit which is formed in a foot massage unit, is located at a portion corresponding to the back of the ankle of the user, is movable forward and backward and includes at least one electrode; and
a control unit for acquiring information on the user's physical condition on the basis of bio-signals obtained from the first bio-signal measurement unit and the second bio-signal measurement unit.

2. The massage apparatus according to claim 1, wherein the first bio-signal measurement unit comprises:
a lower cover put on a housing of the arm massage unit;
at least two magnets fixed on the lower cover and facing a plate;
an upper cover covering the lower cover to protect various components disposed on the lower cover, coupled to the lower cover, and including a palm support having an upward convex shape so that the user can comfortably put the hand thereon;
a first bio-signal measurement electrode inserted into a hole formed in the upper cover; and
the plate made of a metal material, fixed on a cover sheet of the arm massage unit, and located on the upper cover.

3. The massage apparatus according to claim 2, wherein the first bio-signal measurement unit further comprises:
a plurality of LEDs for treatment which are fixed on the lower cover to emit light based on a control signal of the control unit, and
wherein a portion of the upper cover is transparent to transmit light of the plurality of LEDs to the user's hand.

4. The massage apparatus according to claim 2, wherein the first bio-signal measurement unit further comprises:
a heating unit support fixed on the lower cover, located below a heating unit to maintain the shape of the heating unit, and having an upwardly convex shape; and
the heating unit fixed on the heating unit support, formed along the convex surface of the heating unit support, and emitting heat on the basis of the control signal of the control unit.

5. The massage apparatus according to claim 4, wherein the heating unit is located on the plurality of LEDs for treatment, and
wherein the heating unit includes at least one slit not to hide light emitted from the plurality of LEDs for treatment.

6. The massage apparatus according to claim 2, wherein guide rails are formed on the plate in the back-and-forth direction, and the lower cover moves back and forth along the guide rails.

7. The massage apparatus according to claim 2, wherein the upper cover comprises an upper cover fixing portion which is formed along the circumference of the palm support of the upper cover and is flat, and
wherein at least a portion of the upper cover fixing portion is positioned under a cover sheet of the arm massage unit to prevent the upper cover from being separated from the cover sheet of the arm massage unit.

8. The massage apparatus according to claim 1, wherein the second bio-signal measurement unit comprises:
a leg support formed on the housing of the foot massage unit to be located at the back of the leg of the user;
a foot holder coupled to the front of the leg support to support the user's leg, and having a concave shape;
an elastic layer attached to the front of the foot holder; and
a second bio-signal measurement electrode inserted into the hole formed in the cover layer.

9. The massage apparatus according to claim 8, wherein the leg support has a coupling groove into which a coupling protrusion, a spring, and a guide pin included in the foot holder are inserted,
wherein the coupling protrusion of the foot holder has a groove so that a guide ring can be inserted into the groove, and
wherein the leg support and the foot holder are coupled to each other such that the guide pin is inserted into the spring, the guide pin is inserted into the guide ring, the coupling protrusion is inserted into the coupling groove, and the spring is compressed by the guide ring to push the guide ring forward.

10. The massage apparatus according to claim 1, wherein the massage apparatus comprises two first bio-signal measurement units wherein each of the two first bio-signal measurement units includes two first bio-signal measurement electrodes, the massage apparatus comprises two second bio-signal measurement units wherein each of the two second bio-signal measurement units includes two second bio-signal measurement electrodes, and the control unit measures bio-signals of the user by using at least two out of a total of four first bio-signal measurement electrodes and at least two out of a total of four second bio-signal measurement electrodes.

11. The massage apparatus according to claim 1, wherein the control unit measures the user's bio-signals at a predetermined cycle by using the first bio-signal measurement unit and the second bio-signal measurement unit, while the massage apparatus provides a massage to the user according to a massage mode.

12. The massage apparatus according to claim 1, wherein the control unit simultaneously measures body composition information and electrocardiogram information of the user by using the first bio-signal measurement unit and the second bio-signal measurement unit.
